# EUROPEAN PATENT APPLICATION

(11) **EP 3 524 266 A1**
(43) Date of publication of application: **14.08.2019**
(21) Application number: 18155693.7
(22) Date of filing: 08.02.2018
(51) Int. Cl.: A61K 39/12, A61P 33/02, A61K 39/008, A61K 39/00

(54) **VACCINE FOR USE IN THE PREVENTION OR TREATMENT OF LEISHMANIASIS**

(71) Applicant: Universitätsmedizin der Johannes Gutenberg-Universität Mainz, 55131 Mainz (DE)
(72) Inventor: VON STEBUT-BORSCHITZ, Esther, 55122 Mainz (DE); TENZER, Stefan, 55122 Mainz (DE); DIETZE-SCHWONBERG, Kirsten, 55270 Zornheim (DE); SCHERMANN, Anja, 55128 Mainz (DE)
(74) Representative: Patentanwälte Dr. Keller, Schwertfeger Partnerschaft mbB

(57) **Abstract**

The present invention relates to a vaccine comprising a polypeptide or protein selected from the group consisting of *Leishmania major* p54, Q4Q8Z6, Q4Q6L9, Q4QHT2, E9AE98, or an immunologically effective fragment thereof, for use in the prevention or treatment of leishmaniasis. The invention further relates to a pharmaceutical composition comprising such a vaccine.

SEQUENCE LISTING

<110> Universitätsmedizin der Johannes Gutenberg-Universität
Mainz

<120> Vaccines for use in the prevention or treatment of leishmaniasis

<130> Leishmaniasis

<160> 5

<170> PatentIn version 3.5

<210> 1
<211> 9
<212> PRT
<213> Leishmania major

<400> 1

Cys Ala Gln Ala Asn Val Ser Leu Ile
1               5

<210> 2
<211> 807
<212> PRT
<213> Leishmania major

<400> 2

Met Ser Glu Ile Ser Phe Ser Glu Met Ser Leu His Glu Val Pro Leu
1               5                   10                  15
Ala Ser Glu Ala Thr Pro Ser Ser Ala Val Val Leu Gly Gly Arg Thr
            20                  25                  30
Asp Leu Arg Val Gly Asp Phe Val Gly Phe Gln Arg Ile Ser Pro Ser
        35                  40                  45
Lys Glu Trp Lys Leu Ser Leu Gly Lys Val Ile Ala Leu Pro Thr Ala
    50                  55                  60
Leu Thr Val Lys Val Ala His Phe Asp Ala Val Asn Glu Thr Thr Val
65                  70                  75                  80
Thr Gln Asp Pro Ile Ser Glu Gln Met Val Arg Gln Lys Ala Ala Glu
                85                  90                  95
Thr Gln Lys Met Asp Ile Trp His Asn Arg Glu Ala Leu Arg Glu Asn
            100                 105                 110
Leu Ala Gln Glu Ser Ala Lys Glu Lys Ala Ala Thr Gln Arg Leu Leu
        115                 120                 125

Ala Ala Arg Asp Gln Thr Val Thr Arg Gln Met Ala Thr Gly Asp Lys
    130                 135                 140
Val Thr Ala Ser Leu Glu Asn Leu Asp Asn Ala Arg Arg Arg Leu Arg
145                 150                 155                 160
Glu Val Pro Val Lys Glu Trp Gly Ala Leu Arg Ser Ser Tyr Leu Pro
                165                 170                 175
Ser Ala Glu Ile Leu Gly Val Met Arg Ser Val Met Leu Ile Leu Tyr
            180                 185                 190
Glu Asp Ser Val Thr Lys Trp Glu His Ile Gln Glu Val Met His Arg
        195                 200                 205
Gln Asp Phe Met Glu Arg Ile Leu Gly Trp Asp Cys Thr Ala Thr Pro
    210                 215                 220
Met Ser Leu Ser Arg Arg Lys Arg Ile Val Ala Leu Cys Ala Gly Lys
225                 230                 235                 240
Asp Val Glu Gly Ala Pro Ser Lys Lys Leu His Pro Ser Gly Thr Arg
                245                 250                 255

Ser Arg Ser Pro Ser Arg Pro Leu Val Val Ala Ala Lys Ser Thr Asn
            260                 265                 270
Pro Ser Asn Val Val Ile Leu Asp Lys Asn Val Arg Thr Trp Ile Asn
        275                 280                 285
Ala Gln Leu Ser Cys Ser Glu Ala Ala Arg Glu Gln Glu Met Met Ile
    290                 295                 300
Asn Ser Cys Phe Ala Glu Gln Gln Glu Gln Arg Ala Leu Leu Arg Glu
305                 310                 315                 320
Ile Asn Asp Met Arg Val Gly Ile Ser Thr Ile Glu Val Gln Met Leu
                325                 330                 335
Glu Met Lys Asn Ala Ile Leu Gly Ile Asp Asp Ala Pro Arg Pro Ile
            340                 345                 350
Met Pro Leu Asp Ala Tyr Pro Thr Asp Thr Thr Phe Tyr Lys Arg Thr
        355                 360                 365
Tyr Pro Asp Ala Asp Gly Arg Phe Val Gln Glu Ile Ile Leu Arg Glu

    370                 375                 380

Ala Val Ile Leu Asn Phe Gly Pro Ile Ala Glu Glu Asp Ala Glu Gly
385                 390                 395                 400

Tyr Val Arg Leu Asn Ser Ala Gln Val Glu Arg Leu Arg Asp Ala Val
                405                 410                 415

Val Ser Thr Asn Val Arg His Asp Ala Glu Glu Met Glu Glu Leu Leu
            420                 425                 430

Ala Arg Lys Glu Arg Glu Glu Glu Glu Ile Ala Glu Leu Lys Ser Arg
        435                 440                 445

Ile Ser Glu Leu Arg Asn Lys Val Ser Leu Thr Ala Glu Glu Glu Glu
    450                 455                 460

Glu Leu Ala Gln Leu Glu Lys Leu Leu Ala Asp Ala Glu Arg Arg His
465                 470                 475                 480

His Ala Thr Leu Ser Arg Ile Ala Asp Leu Tyr Ala Cys Gly Arg Gly
                485                 490                 495

Ala Arg Glu Ile Thr Leu Ala Ile Lys Arg Pro Glu Phe Arg Tyr Thr
            500                 505                 510
Arg Leu His Cys Lys Met Ser Gly Asp Trp Glu Thr Ile Leu Ser Asp
        515                 520                 525
Ala Glu Arg Tyr Ser Glu Met Ile Ala Ala Phe Cys Asp Asp Val Ser
    530                 535                 540
Thr Leu Leu Asn Ile Pro Ala Ser Tyr Val Leu Asp Ile Asp Thr Cys
545                 550                 555                 560
Cys Gly Ser Leu Leu Ile Asp Phe Thr Val Lys His Ser Gly Asp Leu
                565                 570                 575
Asp Asp Glu Gln Leu Gln Asp Leu Val Asn Lys Gly Cys Phe Ser Ala
            580                 585                 590
Leu Cys Met Phe Tyr Glu Lys Val Thr Phe Lys Lys Thr Ser Pro Leu
        595                 600                 605
Asn Thr Ser Gln Gln Gln Leu Ala Tyr Asp Leu Glu Gln Arg Leu Asn
    610                 615                 620

Gly Pro Ala Pro Ile Ser Gly Met Gly Ile Lys Gln Ile Leu Thr Asp
625                 630                 635                 640
Tyr Tyr Asn Ala Asp Gly Thr Leu Asp Glu Glu Phe Ser Asp Glu Val
                645                 650                 655
Lys Ala His Lys Asp Tyr Arg Lys Ala Leu Ile Thr Ile Pro Pro Leu
            660                 665                 670
Arg Glu Asp Tyr Asp Asp Val Thr Val Arg Gly Pro Phe Glu Gln His
        675                 680                 685
Ala Glu Asp Glu Gly Glu Ala Leu Ser Ala Ala Val Pro Val Ser Ile
    690                 695                 700
Met Ala Ser Ser Val Ala Gly Asn Arg Asn Lys Gly Glu Asp Glu Leu
705                 710                 715                 720
Ala Gly Ala Thr Gln Thr Ser Ile Asp Leu Thr Asp Thr Gly Ala Ala
                725                 730                 735
Thr His Asp Met Thr Glu Ser Ala Pro Ala Met Glu Val His Ser His
            740                 745                 750

Asp Met Asn Leu Ser Tyr Asn Pro Gln Pro Ala Val Asp Ala Ala Ala
        755                 760                 765

Pro Ala Ala Pro Ala Ala Pro Ser Val Ser Ala Pro Ala Glu Asn Val
    770                 775                 780

Gly Gln Pro Gly Val Pro Asn Thr Asp Ala Gly Ser Leu Ser Arg Ser
785                 790                 795                 800

Ser Glu Met Ser Arg Val Asn
                805

<210> 3
<211> 723
<212> PRT
<213> Leishmania major

<400> 3

Met Gly Cys Cys Asn Ser Lys Glu Thr Lys Lys Pro Lys Glu Glu Val
1               5                   10                  15

Leu Gln Arg Glu Ala Gln Tyr Ala Lys Val Ser Ser Glu Thr Asp Ser
            20                  25                  30

Val Glu Glu Gln Pro Asn Ile Glu Ala Arg Val Ala Ser Val Val Lys
        35                  40                  45
Asp Leu Arg Ala Glu Arg Ile Asp Ala Ile Cys Ala His Ser His Ala
    50                  55                  60
Cys Pro Tyr Arg Tyr Asn Ala Cys Thr Val Val Gly Gly Glu Val Lys
65                  70                  75                  80
Phe Tyr Phe Glu Gly Gly Ile Ile Phe Cys Ile Val Lys Glu Gly Ser
                85                  90                  95
Trp Tyr Ile Tyr Asn Asp Ser Leu Asp Tyr Glu Ala His Val Thr Val
            100                 105                 110
Arg Phe Gly Pro Gly Ser Asn Ile Ala Ala Gly Gln Arg Ala Glu Leu
        115                 120                 125
Gly Glu Val Glu Asp Gly Trp Thr Cys Ile Arg Ala Ile Val Tyr Pro
    130                 135                 140
Leu Glu Thr Val His Leu Val Ser Gly Thr Ala Asn Gly Tyr Lys Thr

145                 150                 155                 160

His Ile Thr Ile Lys Pro Leu Asn Glu Gln Tyr Arg His Glu Ala Cys

                165                 170                 175

Ala Ala Ala Asn Gly Thr Ala Glu Ala Glu Thr Glu Ala Val Arg Ser

            180                 185                 190

Leu Val Thr Glu Gly Met Asp Glu Glu Ala Ile Leu His Arg Cys Val

        195                 200                 205

Glu Thr Thr Thr Pro Tyr Val Asp Leu Thr Phe Pro Pro Asn Gly Glu

    210                 215                 220

Ala Val Ala Arg Ala Gly Arg Asp Thr Arg Thr Ile Pro Glu Val Ala

225                 230                 235                 240

Met Met Arg Pro Thr Gln Tyr Leu Leu Asp Asn Asn Cys Leu Ser Val

                245                 250                 255

Asn Asp Ile Cys Gly Pro Val Leu Ala Leu Ser Ile Glu Pro Gly Asn

            260                 265                 270

Leu Gly Asp Ser Trp Phe Met Cys Ala Val Ala Ile Met Ala Glu Asn
        275                 280                 285
Glu Ala Ala Val Arg Ser Ile Phe Ala Gln Gly Ser Pro Ala Glu Lys
    290                 295                 300
Ala Val Gly Ala Tyr Arg Val Leu Val Asn Lys Asn Gly Trp Trp His
305                 310                 315                 320
Thr Leu Ile Met Asp Asp Tyr Leu Pro Thr Phe Asn Arg Met Pro Val
                325                 330                 335
Phe Ala Arg Ser Tyr Asp Asn Pro Ala Glu Val Trp Thr Ser Leu Leu
            340                 345                 350
Gln Lys Ala Tyr Ala Lys Leu His Gly Ser Tyr Ala Ala Ile Thr Gly
        355                 360                 365
Gly Asp Thr Leu Gln Ala Leu Ala Asp Leu Ser Gly Ala Pro Met Cys
    370                 375                 380
Arg Phe Asp Lys Glu Trp Glu Glu Ala Thr Thr Asp Ala Glu Lys Ala
385                 390                 395                 400

Asp Thr Leu Ala Asn Ala Leu Val Gln Phe Ser Arg Ser Gly Ala Cys
                405                 410                 415

Val Val Leu Ser Thr Pro Gly His Asn Ser Glu Ser Tyr Leu Gly Arg
            420                 425                 430

Ser Gln Ala Ser Asp Ala Ala Ala Phe Arg Ala Arg Tyr Lys Lys Val
        435                 440                 445

Gly Leu His Val Gly Tyr Thr Tyr Ser Leu Glu Arg Val Val Met Val
    450                 455                 460

Glu Glu Cys Gly Thr Leu Leu Phe Lys Val Arg Asn Pro Trp Arg Ser
465                 470                 475                 480

Ser Ser Lys Trp Thr Gly Pro Trp Ser Tyr Gly Ser Arg Glu Trp Asp
                485                 490                 495

Glu Asn Gln Asp Ala Cys Leu Leu Cys Gly Ala Gln Lys Asp Pro Gln
            500                 505                 510

Asp Gly Ser Phe Trp Met Cys Trp Asp Asp Ala Ser Arg Tyr Phe Asp
        515                 520                 525

Gly Gly Gly Val Leu Phe Ala Asn Pro Asp Ala Val Asp Tyr Arg Val
    530                 535                 540

Lys Gly Val Phe Cys Lys Thr Ile Pro Ser Ala Val Leu Glu Ile Thr
545                 550                 555                 560

Ala Ser Glu Ser Thr Gln Val Phe Phe Thr Leu Ser Gln Pro Asp Lys
                565                 570                 575

Arg Gly Val Asp Arg Lys Glu Gly Ala Ala Leu Phe Ala Pro Ile Met
            580                 585                 590

Leu Thr Val Ser Lys Glu Glu Asp Gly Val Gln Arg Val Gln Lys Asn
        595                 600                 605

Thr Ser Trp Asn Pro Thr Met Pro Ser Glu Glu Phe Asn Phe Val Val
    610                 615                 620

Gly Arg Asp Val Ser Met Trp Phe Thr Leu Gly Ala Glu Glu Lys Tyr
625                 630                 635                 640

Leu Val Val Pro Arg Ile His Ser Lys Gly Val Lys Ser Asp Tyr Asp

                645                 650                 655

Arg Pro Tyr Val Ile Ser Ile Met Ser Thr Asp Lys Leu Glu Gly Asn
            660                 665                 670

Val Arg Val Glu Ala Lys His Ile His Ser Asp Ser Lys Val Phe Thr
        675                 680                 685

Asn Tyr Ile Ala Tyr Asp Ser Glu Glu Leu Pro Ser Val Lys Val Glu
    690                 695                 700

Asn Gln Val Arg Leu Pro Gly Lys Ala Pro Val Thr Tyr Val Ser Ala
705                 710                 715                 720

Thr Val Ile

<210> 4
<211> 149
<212> PRT
<213> Leishmania major

<400> 4

Met Ala Asp Gln Leu Ser Asn Glu Gln Ile Ser Glu Phe Lys Glu Ala

1               5                   10                  15

Phe Ser Leu Phe Asp Lys Asp Gly Asp Gly Thr Ile Thr Thr Lys Glu

            20                  25                  30

Leu Gly Thr Val Met Arg Ser Leu Gly Gln Asn Pro Thr Glu Ala Glu

        35                  40                  45

Leu Gln Asp Met Ile Asn Glu Val Asp Gln Asp Gly Ser Gly Thr Ile

    50                  55                  60

Asp Phe Pro Glu Phe Leu Thr Leu Met Ala Arg Lys Met Gln Asp Ser

65                  70                  75                  80

Asp Ser Glu Glu Glu Ile Lys Glu Ala Phe Arg Val Phe Asp Lys Asp

                85                  90                  95

Gly Asn Gly Phe Ile Ser Ala Ala Glu Leu Arg His Val Met Thr Asn

            100                 105                 110

Leu Gly Glu Lys Leu Thr Asp Glu Glu Val Asp Glu Met Ile Arg Glu

        115                 120                 125

Ala Asp Val Asp Gly Asp Gly Gln Ile Asn Tyr Glu Glu Phe Val Lys
    130                 135                 140

Met Met Met Ser Lys
145

<210> 5
<211> 453
<212> PRT
<213> Leishmania major

<400> 5

Met Ala Thr Ile Met Ser Glu His Val Val Lys Met Ala Lys Glu Phe
1               5                   10                  15

Val Asn Phe Met Asn Gln Ala Ile Thr Pro Phe His Ala Val Gln Thr
            20                  25                  30

Val Ala Lys Met Leu Lys Asp Ala Gly Tyr Thr Gln Leu His Glu Glu
        35                  40                  45

Lys Val Trp Pro Glu Leu Thr Pro Gly Gly Lys Tyr Tyr Leu Met Arg
    50                  55                  60

Asn Gly Thr Ser Ile Ile Ala Phe Ser Val Gly Gly Lys Phe Asp Pro
65                  70                  75                  80

Met Asn Gly Val Lys Ile Val Gly Ala His Thr Asp Ser Pro Asn Phe
                85                  90                  95

Leu Leu Lys Pro Arg Thr Lys Ser Thr Ala Ala Asp Tyr Glu Arg Val
            100                 105                 110

Ala Val Gln Cys Tyr Gly Gly Gly Leu Trp His Ser Trp Phe Asp Arg
        115                 120                 125

Asp Leu Thr Val Ala Gly Arg Val Met Ile Ser Arg Glu Arg Leu Glu
    130                 135                 140

Gln Lys Ile Ile Lys Ile Asp Lys Pro Ile Met Arg Ile Pro Asn Leu
145                 150                 155                 160

Ala Ile His Leu Thr Ser Ala Lys Asp Arg Glu Ser Phe Ser Pro Asn
                165                 170                 175

Lys Glu Ser His Leu Ile Pro Ile Ile Ser Thr Gln Ile Ala Ala Arg
            180                 185                 190

Ile Ala Glu Cys Asp Asp Lys Asp Ala Ser Asn Pro Asn His Cys Val
        195                 200                 205
Ser Leu Ile Lys Ala Ile Ala Ser Val Ala Glu Cys Asn Pro Asp Glu
    210                 215                 220
Ile Val Asp Phe Asp Leu Ser Val Ile Asp Thr Gln Pro Ala Val Ile
225                 230                 235                 240
Gly Gly Ile His Asp Glu Phe Ile Phe Ser Pro Arg Leu Asp Asn Leu
                245                 250                 255
Ile Ser Cys Tyr Cys Ala Val Lys Ala Ile Ile Glu Ala Asp Ala Leu
            260                 265                 270
Glu Asn Asp Thr Met Met Arg Met Val Cys Leu Phe Asp His Glu Glu
        275                 280                 285
Cys Gly Ser Ser Ser Ser Gln Gly Ala Ala Gly Ser Leu Val Pro Asp
    290                 295                 300
Val Ile Glu His Ile Val Ser Asn Lys Thr Leu Arg Ala Thr Leu Val
305                 310                 315                 320

Ala Asn Ser Phe Leu Leu Ser Val Asp Gly Ala His Gly Cys His Pro
                325                 330                 335
Asn Tyr Ala Asp Lys His Glu Asn Ala His Arg Pro Ala Leu His Gly
            340                 345                 350
Gly Pro Val Ile Lys Tyr Asn Ala Asn Ala Arg Tyr Ala Thr Asn Gly
        355                 360                 365
Leu Thr Ala Ala Val Val Lys Asp Val Ala Lys Lys Ala Ala Ile Pro
    370                 375                 380
Ile Gln Glu Phe Val Val Arg Asn Asp Ser Pro Cys Gly Ser Thr Ile
385                 390                 395                 400
Gly Pro Ile Leu Ser Ser Leu Ser Gly Ile Lys Thr Ala Asp Ile Gly
                405                 410                 415
Asn Pro Met Ile Ser Met His Ser Ile Arg Glu Met Cys Gly Thr Val
            420                 425                 430
Asp Val Tyr Tyr Met Thr Lys Leu Ile Glu Ser Phe Phe Ile Asn Tyr

        435                 440                 445

Gln Asn Pro His Glu

    450

SEQUENCE LISTING

<110>  Universitätsmedizin der Johannes Gutenberg-Universität
       Mainz

<120>  Vaccines for use in the prevention or treatment of leishmaniasis

<130>  Leishmaniasis

<160>  5

<170>  PatentIn version 3.5

<210>  1
<211>  9
<212>  PRT
<213>  Leishmania major

<400>  1

Cys Ala Gln Ala Asn Val Ser Leu Ile
1               5


<210>  2
<211>  807
<212>  PRT
<213>  Leishmania major

<400>  2

Met Ser Glu Ile Ser Phe Ser Glu Met Ser Leu His Glu Val Pro Leu
1               5                   10                  15


Ala Ser Glu Ala Thr Pro Ser Ser Ala Val Val Leu Gly Gly Arg Thr
            20                  25                  30


Asp Leu Arg Val Gly Asp Phe Val Gly Phe Gln Arg Ile Ser Pro Ser
        35                  40                  45


Lys Glu Trp Lys Leu Ser Leu Gly Lys Val Ile Ala Leu Pro Thr Ala
    50                  55                  60


Leu Thr Val Lys Val Ala His Phe Asp Ala Val Asn Glu Thr Thr Val
65                  70                  75                  80


Thr Gln Asp Pro Ile Ser Glu Gln Met Val Arg Gln Lys Ala Ala Glu
                85                  90                  95


Thr Gln Lys Met Asp Ile Trp His Asn Arg Glu Ala Leu Arg Glu Asn
            100                 105                 110


Leu Ala Gln Glu Ser Ala Lys Glu Lys Ala Ala Thr Gln Arg Leu Leu
        115                 120                 125

Ala Ala Arg Asp Gln Thr Val Thr Arg Gln Met Ala Thr Gly Asp Lys
    130                 135                 140

Val Thr Ala Ser Leu Glu Asn Leu Asp Asn Ala Arg Arg Arg Leu Arg
145                 150                 155                 160

Glu Val Pro Val Lys Glu Trp Gly Ala Leu Arg Ser Ser Tyr Leu Pro
                165                 170                 175

Ser Ala Glu Ile Leu Gly Val Met Arg Ser Val Met Leu Ile Leu Tyr
            180                 185                 190

Glu Asp Ser Val Thr Lys Trp Glu His Ile Gln Glu Val Met His Arg
        195                 200                 205

Gln Asp Phe Met Glu Arg Ile Leu Gly Trp Asp Cys Thr Ala Thr Pro
    210                 215                 220

Met Ser Leu Ser Arg Arg Lys Arg Ile Val Ala Leu Cys Ala Gly Lys
225                 230                 235                 240

Asp Val Glu Gly Ala Pro Ser Lys Lys Leu His Pro Ser Gly Thr Arg
                245                 250                 255

Ser Arg Ser Pro Ser Arg Pro Leu Val Val Ala Ala Lys Ser Thr Asn
            260                 265                 270

Pro Ser Asn Val Val Ile Leu Asp Lys Asn Val Arg Thr Trp Ile Asn
        275                 280                 285

Ala Gln Leu Ser Cys Ser Glu Ala Ala Arg Glu Gln Glu Met Met Ile
    290                 295                 300

Asn Ser Cys Phe Ala Glu Gln Gln Glu Gln Arg Ala Leu Leu Arg Glu
305                 310                 315                 320

Ile Asn Asp Met Arg Val Gly Ile Ser Thr Ile Glu Val Gln Met Leu
                325                 330                 335

Glu Met Lys Asn Ala Ile Leu Gly Ile Asp Asp Ala Pro Arg Pro Ile
            340                 345                 350

Met Pro Leu Asp Ala Tyr Pro Thr Asp Thr Thr Phe Tyr Lys Arg Thr
        355                 360                 365

Tyr Pro Asp Ala Asp Gly Arg Phe Val Gln Glu Ile Ile Leu Arg Glu
    370                 375                 380

Ala Val Ile Leu Asn Phe Gly Pro Ile Ala Glu Glu Asp Ala Glu Gly
385                 390                 395                 400

Tyr Val Arg Leu Asn Ser Ala Gln Val Glu Arg Leu Arg Asp Ala Val
                405                 410                 415

Val Ser Thr Asn Val Arg His Asp Ala Glu Glu Met Glu Glu Leu Leu
            420                 425                 430

Ala Arg Lys Glu Arg Glu Glu Glu Glu Ile Ala Glu Leu Lys Ser Arg
        435                 440                 445

Ile Ser Glu Leu Arg Asn Lys Val Ser Leu Thr Ala Glu Glu Glu Glu
    450                 455                 460

Glu Leu Ala Gln Leu Glu Lys Leu Leu Ala Asp Ala Glu Arg Arg His
465                 470                 475                 480

His Ala Thr Leu Ser Arg Ile Ala Asp Leu Tyr Ala Cys Gly Arg Gly
                485                 490                 495

Ala Arg Glu Ile Thr Leu Ala Ile Lys Arg Pro Glu Phe Arg Tyr Thr
            500                 505                 510

Arg Leu His Cys Lys Met Ser Gly Asp Trp Glu Thr Ile Leu Ser Asp
        515                 520                 525

Ala Glu Arg Tyr Ser Glu Met Ile Ala Ala Phe Cys Asp Asp Val Ser
    530                 535                 540

Thr Leu Leu Asn Ile Pro Ala Ser Tyr Val Leu Asp Ile Asp Thr Cys
545                 550                 555                 560

Cys Gly Ser Leu Leu Ile Asp Phe Thr Val Lys His Ser Gly Asp Leu
                565                 570                 575

Asp Asp Glu Gln Leu Gln Asp Leu Val Asn Lys Gly Cys Phe Ser Ala
            580                 585                 590

Leu Cys Met Phe Tyr Glu Lys Val Thr Phe Lys Lys Thr Ser Pro Leu
        595                 600                 605

Asn Thr Ser Gln Gln Gln Leu Ala Tyr Asp Leu Glu Gln Arg Leu Asn
    610                 615                 620

Gly Pro Ala Pro Ile Ser Gly Met Gly Ile Lys Gln Ile Leu Thr Asp
625                 630                 635                 640

Tyr Tyr Asn Ala Asp Gly Thr Leu Asp Glu Glu Phe Ser Asp Glu Val
                645                 650                 655

Lys Ala His Lys Asp Tyr Arg Lys Ala Leu Ile Thr Ile Pro Pro Leu
            660                 665                 670

Arg Glu Asp Tyr Asp Asp Val Thr Val Arg Gly Pro Phe Glu Gln His
        675                 680                 685

Ala Glu Asp Glu Gly Glu Ala Leu Ser Ala Ala Val Pro Val Ser Ile
    690                 695                 700

Met Ala Ser Ser Val Ala Gly Asn Arg Asn Lys Gly Glu Asp Glu Leu
705                 710                 715                 720

Ala Gly Ala Thr Gln Thr Ser Ile Asp Leu Thr Asp Thr Gly Ala Ala
                725                 730                 735

Thr His Asp Met Thr Glu Ser Ala Pro Ala Met Glu Val His Ser His
            740                 745                 750

Asp Met Asn Leu Ser Tyr Asn Pro Gln Pro Ala Val Asp Ala Ala Ala
        755                 760                 765

Pro Ala Ala Pro Ala Ala Pro Ser Val Ser Ala Pro Ala Glu Asn Val
    770                 775                 780

Gly Gln Pro Gly Val Pro Asn Thr Asp Ala Gly Ser Leu Ser Arg Ser
785                 790                 795                 800

Ser Glu Met Ser Arg Val Asn
                805

<210>  3
<211>  723
<212>  PRT
<213>  Leishmania major

<400>  3

Met Gly Cys Cys Asn Ser Lys Glu Thr Lys Lys Pro Lys Glu Glu Val
1               5                   10                  15

Leu Gln Arg Glu Ala Gln Tyr Ala Lys Val Ser Ser Glu Thr Asp Ser
            20                  25                  30

Val Glu Glu Gln Pro Asn Ile Glu Ala Arg Val Ala Ser Val Val Lys
        35                  40                  45

Asp Leu Arg Ala Glu Arg Ile Asp Ala Ile Cys Ala His Ser His Ala
    50                  55                  60

Cys Pro Tyr Arg Tyr Asn Ala Cys Thr Val Val Gly Gly Glu Val Lys
65                  70                  75                  80

Phe Tyr Phe Glu Gly Gly Ile Ile Phe Cys Ile Val Lys Glu Gly Ser
                85                  90                  95

Trp Tyr Ile Tyr Asn Asp Ser Leu Asp Tyr Glu Ala His Val Thr Val
            100                 105                 110

Arg Phe Gly Pro Gly Ser Asn Ile Ala Ala Gly Gln Arg Ala Glu Leu
        115                 120                 125

Gly Glu Val Glu Asp Gly Trp Thr Cys Ile Arg Ala Ile Val Tyr Pro
    130                 135                 140

Leu Glu Thr Val His Leu Val Ser Gly Thr Ala Asn Gly Tyr Lys Thr
145                 150                 155                 160

His Ile Thr Ile Lys Pro Leu Asn Glu Gln Tyr Arg His Glu Ala Cys
                165                 170                 175

Ala Ala Ala Asn Gly Thr Ala Glu Ala Glu Thr Glu Ala Val Arg Ser
            180                 185                 190

Leu Val Thr Glu Gly Met Asp Glu Glu Ala Ile Leu His Arg Cys Val
        195                 200                 205

Glu Thr Thr Thr Pro Tyr Val Asp Leu Thr Phe Pro Pro Asn Gly Glu
    210                 215                 220

Ala Val Ala Arg Ala Gly Arg Asp Thr Arg Thr Ile Pro Glu Val Ala
225                 230                 235                 240

Met Met Arg Pro Thr Gln Tyr Leu Leu Asp Asn Asn Cys Leu Ser Val
                245                 250                 255

Asn Asp Ile Cys Gly Pro Val Leu Ala Leu Ser Ile Glu Pro Gly Asn
            260                 265                 270

Leu Gly Asp Ser Trp Phe Met Cys Ala Val Ala Ile Met Ala Glu Asn
        275                 280                 285

Glu Ala Ala Val Arg Ser Ile Phe Ala Gln Gly Ser Pro Ala Glu Lys

290                 295                 300

Ala Val Gly Ala Tyr Arg Val Leu Val Asn Lys Asn Gly Trp Trp His
305                 310                 315                 320

Thr Leu Ile Met Asp Asp Tyr Leu Pro Thr Phe Asn Arg Met Pro Val
                325                 330                 335

Phe Ala Arg Ser Tyr Asp Asn Pro Ala Glu Val Trp Thr Ser Leu Leu
            340                 345                 350

Gln Lys Ala Tyr Ala Lys Leu His Gly Ser Tyr Ala Ala Ile Thr Gly
        355                 360                 365

Gly Asp Thr Leu Gln Ala Leu Ala Asp Leu Ser Gly Ala Pro Met Cys
    370                 375                 380

Arg Phe Asp Lys Glu Trp Glu Glu Ala Thr Thr Asp Ala Glu Lys Ala
385                 390                 395                 400

Asp Thr Leu Ala Asn Ala Leu Val Gln Phe Ser Arg Ser Gly Ala Cys
                405                 410                 415

Val Val Leu Ser Thr Pro Gly His Asn Ser Glu Ser Tyr Leu Gly Arg
            420                 425                 430

Ser Gln Ala Ser Asp Ala Ala Ala Phe Arg Ala Arg Tyr Lys Lys Val
        435                 440                 445

Gly Leu His Val Gly Tyr Thr Tyr Ser Leu Glu Arg Val Val Met Val
    450                 455                 460

Glu Glu Cys Gly Thr Leu Leu Phe Lys Val Arg Asn Pro Trp Arg Ser
465                 470                 475                 480

Ser Ser Lys Trp Thr Gly Pro Trp Ser Tyr Gly Ser Arg Glu Trp Asp
                485                 490                 495

Glu Asn Gln Asp Ala Cys Leu Leu Cys Gly Ala Gln Lys Asp Pro Gln
            500                 505                 510

Asp Gly Ser Phe Trp Met Cys Trp Asp Asp Ala Ser Arg Tyr Phe Asp
        515                 520                 525

Gly Gly Gly Val Leu Phe Ala Asn Pro Asp Ala Val Asp Tyr Arg Val
    530                 535                 540

Lys Gly Val Phe Cys Lys Thr Ile Pro Ser Ala Val Leu Glu Ile Thr
545                 550                 555                 560

Ala Ser Glu Ser Thr Gln Val Phe Phe Thr Leu Ser Gln Pro Asp Lys
                565                 570                 575

Arg Gly Val Asp Arg Lys Glu Gly Ala Ala Leu Phe Ala Pro Ile Met
            580                 585                 590

Leu Thr Val Ser Lys Glu Glu Asp Gly Val Gln Arg Val Gln Lys Asn
        595                 600                 605

Thr Ser Trp Asn Pro Thr Met Pro Ser Glu Glu Phe Asn Phe Val Val
    610                 615                 620

Gly Arg Asp Val Ser Met Trp Phe Thr Leu Gly Ala Glu Glu Lys Tyr
625                 630                 635                 640

Leu Val Val Pro Arg Ile His Ser Lys Gly Val Lys Ser Asp Tyr Asp
                645                 650                 655

Arg Pro Tyr Val Ile Ser Ile Met Ser Thr Asp Lys Leu Glu Gly Asn
            660                 665                 670

Val Arg Val Glu Ala Lys His Ile His Ser Asp Ser Lys Val Phe Thr
        675                 680                 685

Asn Tyr Ile Ala Tyr Asp Ser Glu Glu Leu Pro Ser Val Lys Val Glu
    690                 695                 700

Asn Gln Val Arg Leu Pro Gly Lys Ala Pro Val Thr Tyr Val Ser Ala
705                 710                 715                 720

Thr Val Ile

<210>  4
<211>  149
<212>  PRT
<213>  Leishmania major

<400>  4

Met Ala Asp Gln Leu Ser Asn Glu Gln Ile Ser Glu Phe Lys Glu Ala
1               5                   10                  15

Phe Ser Leu Phe Asp Lys Asp Gly Asp Gly Thr Ile Thr Thr Lys Glu
            20                  25                  30

Leu Gly Thr Val Met Arg Ser Leu Gly Gln Asn Pro Thr Glu Ala Glu
        35                  40                  45

Leu Gln Asp Met Ile Asn Glu Val Asp Gln Asp Gly Ser Gly Thr Ile
    50                  55                  60

Asp Phe Pro Glu Phe Leu Thr Leu Met Ala Arg Lys Met Gln Asp Ser
65                  70                  75                  80

Asp Ser Glu Glu Glu Ile Lys Glu Ala Phe Arg Val Phe Asp Lys Asp
                85                  90                  95

Gly Asn Gly Phe Ile Ser Ala Ala Glu Leu Arg His Val Met Thr Asn
            100                 105                 110

Leu Gly Glu Lys Leu Thr Asp Glu Glu Val Asp Glu Met Ile Arg Glu
        115                 120                 125

Ala Asp Val Asp Gly Asp Gly Gln Ile Asn Tyr Glu Glu Phe Val Lys
    130                 135                 140

Met Met Met Ser Lys
145

<210>  5
<211>  453
<212>  PRT
<213>  Leishmania major

<400>  5

Met Ala Thr Ile Met Ser Glu His Val Val Lys Met Ala Lys Glu Phe
1               5                   10                  15

Val Asn Phe Met Asn Gln Ala Ile Thr Pro Phe His Ala Val Gln Thr
            20                  25                  30

Val Ala Lys Met Leu Lys Asp Ala Gly Tyr Thr Gln Leu His Glu Glu
        35                  40                  45

Lys Val Trp Pro Glu Leu Thr Pro Gly Gly Lys Tyr Tyr Leu Met Arg
    50                  55                  60

Asn Gly Thr Ser Ile Ile Ala Phe Ser Val Gly Gly Lys Phe Asp Pro
65                  70                  75                  80

Met Asn Gly Val Lys Ile Val Gly Ala His Thr Asp Ser Pro Asn Phe
                85                  90                  95

Leu Leu Lys Pro Arg Thr Lys Ser Thr Ala Ala Asp Tyr Glu Arg Val
            100                 105                 110

Ala Val Gln Cys Tyr Gly Gly Gly Leu Trp His Ser Trp Phe Asp Arg
        115                 120                 125

Asp Leu Thr Val Ala Gly Arg Val Met Ile Ser Arg Glu Arg Leu Glu
    130                 135                 140

Gln Lys Ile Ile Lys Ile Asp Lys Pro Ile Met Arg Ile Pro Asn Leu
145                 150                 155                 160

Ala Ile His Leu Thr Ser Ala Lys Asp Arg Glu Ser Phe Ser Pro Asn
                165                 170                 175

Lys Glu Ser His Leu Ile Pro Ile Ile Ser Thr Gln Ile Ala Ala Arg
            180                 185                 190

Ile Ala Glu Cys Asp Asp Lys Asp Ala Ser Asn Pro Asn His Cys Val
        195                 200                 205

Ser Leu Ile Lys Ala Ile Ala Ser Val Ala Glu Cys Asn Pro Asp Glu
    210                 215                 220

Ile Val Asp Phe Asp Leu Ser Val Ile Asp Thr Gln Pro Ala Val Ile
225                 230                 235                 240

Gly Gly Ile His Asp Glu Phe Ile Phe Ser Pro Arg Leu Asp Asn Leu
                245                 250                 255

Ile Ser Cys Tyr Cys Ala Val Lys Ala Ile Ile Glu Ala Asp Ala Leu
            260                 265                 270

Glu Asn Asp Thr Met Met Arg Met Val Cys Leu Phe Asp His Glu Glu
        275                 280                 285

Cys Gly Ser Ser Ser Ser Gln Gly Ala Ala Gly Ser Leu Val Pro Asp
    290                 295                 300

Val Ile Glu His Ile Val Ser Asn Lys Thr Leu Arg Ala Thr Leu Val
305                 310                 315                 320

Ala Asn Ser Phe Leu Leu Ser Val Asp Gly Ala His Gly Cys His Pro
                325                 330                 335

Asn Tyr Ala Asp Lys His Glu Asn Ala His Arg Pro Ala Leu His Gly
            340                 345                 350

Gly Pro Val Ile Lys Tyr Asn Ala Asn Ala Arg Tyr Ala Thr Asn Gly
        355                 360                 365

Leu Thr Ala Ala Val Val Lys Asp Val Ala Lys Lys Ala Ala Ile Pro
    370                 375                 380

Ile Gln Glu Phe Val Val Arg Asn Asp Ser Pro Cys Gly Ser Thr Ile
385                 390                 395                 400

Gly Pro Ile Leu Ser Ser Leu Ser Gly Ile Lys Thr Ala Asp Ile Gly
                405                 410                 415

Asn Pro Met Ile Ser Met His Ser Ile Arg Glu Met Cys Gly Thr Val
            420                 425                 430

Asp Val Tyr Tyr Met Thr Lys Leu Ile Glu Ser Phe Phe Ile Asn Tyr
        435                 440                 445

Gln Asn Pro His Glu
    450

## Description

The present invention relates to a vaccine, or an immunologically effective fragment thereof, for use in the prevention or treatment of leishmaniasis.

Leishmaniasis is a disease caused by *Leishmania* parasites transmitted by the bite of an infected sand fly (*Phlebotominae*). The clinical picture of the disease ranges from skin lesions to severe disfigurement and fatal systemic infection. The disease can be present in humans and mammalian animals in three different ways in form of cutaneous, mucocutaneous or visceral leishmaniasis. A cutaneous leishmaniasis results in the formation of skin ulcers and accounts for more than 50% of new cases of leishmaniasis. The mucocutaneous form is characterized by the late development of metastatic lesions that can lead to the destruction of the mucous membrane. The visceral form of leishmaniasis (also known as kala-azar) is the most severe and starts with skin ulcers and then later results in fatal syndrome, along with fever, low red blood cells and alterations of the spleen and liver.

Infections in humans are caused by different species of *Leishmania.* There are currently 12 Million people affected worldwide and leishmaniasis threatens 350 million people in 88 countries. The disease also occurs in animals such as in dogs, horses or cats. Among animal leishmaniasis, canine leishmaniasis is well studied, and predominantly caused by *Leishmania* (*L*.) *donovani* and *L. infantum.* Usually, also canine transmission is directly mediated from sand fly to dog. However, there are also cases of *L. infantum* transmission wherein the infection has been proven from dog to dog by a direct contamination with blood and secretions.

One major *Leishmania* type that causes cutaneous leishmaniasis is the protozoan species *L*. *major.* Healing of *L. major* infections is related with IFNγ secretion by CD4⁺ and CD8⁺ T cells. The development of a successful vaccine to prevent leishmaniasis has been a goal for almost a century, but currently no such vaccine exists (Lukasz Kedzierksi, Leishmaiasis Vaccine: Where are We Today?, J Glob infect Dis. 2010 2(2): 177-185). It is also unclear which proteins or peptides are essentially required for parasite clearance and therefore can be used as vaccines. Current treatment is based on chemotherapy, which relies on a handful of drugs with serious limitations such as high cost and toxicity, difficult form of administration and lack of efficiency in endemic areas. The pentavalent antimonials such as sodium stibugluconate and meglumine antimoniate have been recommended for the treatment of leishmaniasis for over 70 years. Resistance to this class of drug is increasing and the efficiency is dropping. Second line drugs used in treatment of leishmaniasis include aromatic diamidines (Pentamidine) and amphotericin B, but similarly to the pentavalent antimonials, these drugs are toxic and come along with severe side effects. Therefore, *Leishmania* vaccine development has proven to be a difficult and challenging task, which is almost hampered by inadequate knowledge of parasite pathogenesis and the complexity of immune responses needed for protection.

Most of the vaccine studies concentrate on the second generation vaccines consisting of recombinant proteins, polypeptides, DNA vaccines or dendritic cells loaded with peptides derived from leishmanial antigens. A variety of different molecules has been tested to date, and these included antigens such as surface expressed glycoprotein leishmanolysin (gp63) delivered by a plethora of immunization regimens, however, promising findings from animal models were overshadowed by mostly negative T cell responses in humans (Russo DM, Burns JM Jr, Carvalho EM, Armitage RJ, Grabstein KH, Button LL, et al. Human T cell responses to gp63, a surface antigen of Leishmania. J Immunol 1991 ;147:3575-80.). Another vaccine candidate has been a GPI-anchored membrane protein gp46 or Parasite Surface Antigen 2 (PSA-2), that belongs to a gene family present in all *Leishmania* species except *L. braziliensis* (McMahon-Pratt D, Traub-Cseko Y, Lohman KL, Rogers DD, Beverley SM. Loss of the GP46/M-2 surface membrane glycoprotein gene family in the Leishmania braziliensis complex. MolBiochem Parasitol 1992; 50: 151-60.).

Immunization with the native polypeptides derived from promastigotes protected mice against infection (Handman E, Symons FM, Baldwin TM, Curtis JM, Scheerlinck JP. Protective vaccination with promastigote surface antigen 2 from Leishmania major is mediated by a TH1 type of immune response. Infect Immun 1995;63:4261-7.), but vaccination with a recombinant protein derived from either promastigotes or amastigotes showed lack of protective efficacy (Sjolander A, Baldwin TM, Curtis JM, Bengtsson KL, Handman E. Vaccination with recombinant Parasite Surface Antigen 2 from Leishmania major induces a Th1 type of immune response but does not protect against infection. Vaccine 1998;16:2077-84.). Similarly, DNA vaccination conferred protection in mice when used as either prophylactic (Sjolander A, Baldwin TM, Curtis JM, Handman E. Induction of a Th1 immune response and simultaneous lack of activation of a Th2 response are required for generation of immunity to leishmaniasis. J Immunol 1998;160:3949-57.) or therapeutic vaccines (Handman E, Noormohammadi AH, Curtis JM, Baldwin T, Sjolander A. Therapy of murine cutaneous leishmaniasis by DNA vaccination. Vaccine 2000;18:3011-7.).

Another extensively tested antigen is the *Leishmania* homologue for receptors of activated C kinase (LACK) that is expressed throughout leishmanial life cycle (Mougneau E, Altare F, Wakil AE, Zheng S, Coppola T, Wang ZE, et al. Expression cloning of a protective Leishmania antigen. Science 1995;268:563-6.). Immunization with LACK appears to promote the expansion of IL-4 secreting T cells skewing the response towards detrimental Th2 responses (Launois P, Maillard I, Pingel S, Swihart KG, Xenarios I, Acha-Orbea H, et al. IL-4 rapidly produced by V beta 4 V alpha 8 CD4+ T cells instructs Th2 development and susceptibility to Leishmania major in BALB/c mice. Immunity 1997;6:541-9.), however, susceptible BALB/c mice immunized with LACK had the ability to control a subsequent infection with *L. major* (Julia V, Rassoulzadegan M, Glaichenhaus N. Resistance to Leishmania major induced by tolerance to a single antigen. Science 1996;274:421-3.). To date, the protective efficacy of LACK has been mainly demonstrated in the *L. major* model, and LACK failed to protect against visceral leishmaniasis (Melby PC, Yang J, Zhao W, Perez LE, Cheng J. Leishmania donovanip36(LACK) DNA vaccine is highly immunogenic but not protective against experimental visceral leishmaniasis. Infect Immun 2001 ;69:4719-25.).

Several other antigens from different species have been tested in animal models. These include amastigote cysteine proteases (CP) (Rafati S, Nakhaee A, Taheri T, Taslimi Y, Darabi H, Eravani D, et al. Protective vaccination against experimental canine visceral leishmaniasis using a combination of DNA and protein immunization with cysteine proteinases type I and II of L. infantum. Vaccine 2005;23:3716-25.), cysteine proteinase A2 and amastigote membrane proteins P4 and P8 (Soong L, Duboise SM, Kima P, McMahon-Pratt D. Leishmania pifanoi amastigote antigens protect mice against cutaneous leishmaniasis. Infect Immun 1995;63:3559-66.), kinetoplastid membrane protein-11 (KMP-11) (Basu R, Bhaumik S, Basu JM, Naskar K, De T, Roy S. Kinetoplastid membrane protein-11 DNA vaccination induces complete protection against both pentavalent antimonial-sensitive and -resistant strains of Leishmania donovani that correlates with inducible nitric oxide synthase activity and IL-4 generation: evidence for mixed Th1- and Th2-like responses in visceral leishmaniasis. J Immunol 2005;174:7160-71.), amastigote LCR1 (Streit JA, Recker TJ, Donelson JE, Wilson ME. BCG expressing LCR1 of Leishmania chagasi induces protective immunity in susceptible mice. Exp Parasitol 2000;94:33-41.), hydrophilic acylated surface protein B1 (HASPB1) (Stager S, Smith DF, Kaye PM. Immunization with a recombinant stage-regulated surface protein from Leishmania donovani induces protection against visceral leishmaniasis. J Immunol 2000;165:7064-71.), leishmanial antigen ORFF (Tewary P, Jain M, Sahani MH, Saxena S, Madhubala R. A heterologous prime-boost vaccination regimen using ORFF DNA and recombinant ORFF protein confers protective immunity against experimental visceral leishmaniasis. J Infect Dis 2005;191:2130-7.), acidic ribosomal protein P0 (Iborra S, Soto M, Carrion J, Nieto A, Fernandez E, Alonso C, et al. The Leishmania infantum acidic ribosomal protein P0 administered as a DNA vaccine confers protective immunity to Leishmania major infection in BALB/c mice. Infect Immun 2003;71:6562-72.), paraflagellar rod protein 2 (PRP-2) (Saravia NG, Hazbon MH, Osorio Y, Valderrama L, Walker J, Santrich C, et al. Protective immunogenicity of the paraflagellar rod protein 2 of Leishmania mexicana. Vaccine 2005;23:984-95.), 36 kDa nucleoside hydrolase (NH36), a main component of the fucose-mannose ligand (Aguilar-Be I, da Silva Zardo R, Paraguai de Souza E, Borja-Cabrera GP, Rosado-Vallado M, Mut-Martin M, et al. Cross-protective efficacy of a prophylactic Leishmania donovani DNA vaccine against visceral and cutaneous murine leishmaniasis. Infect Immun 2005;73:812-9.), and proteophosphoglycan (PPG) (Samant M, Gupta R, Kumari S, Misra P, Khare P, Kushawaha PK, et al. Immunization with the DNA-encoding N-terminal domain of proteophosphoglycan of Leishmania donovani generates Th1-type immunoprotective response against experimental visceral leishmaniasis. J Immunol 2009;183:470-9.). In addition, molecules such as ATP synthase alpha chain, beta-tubulin and heat shock 70-related protein 1 precursor have been recently identified as novel vaccine candidates (Bhowmick S, Ali N. Identification of novel Leishmania donovani antigens that help define correlates of vaccine-mediated protection in visceral leishmaniasis. PLoS One 2009;4:e5820.).

To date, only one second generation vaccine, Leish-111f, has been assessed in clinical trials. Leish-111f is a single polyprotein composed of three fused molecules; the *L. major* homologue of eukaryotic thiol-specific antioxidant (TSA), the *L. major* stress-inducible protein-1 (LmSTI1) and the *L. braziliensis* elongation and initiation factor (LeIF) (Coler RN, Reed SG. Second-generation vaccines against leishmaniasis. Trends Parasitol 2005;21:244-9.). Initial immunization trials in mice demonstrated that Leish-111f was able to protect mice against *L*. *major* and *L. amazonensis* infection (Skeiky YA, Coler RN, Brannon M, Stromberg E, Greeson K, Crane RT, et al. Protective efficacy of a tandemly linked, multi-subunit recombinant leishmanial vaccine (Leish-111f) formulated in MPL adjuvant. Vaccine 2002;20:3292-303.). There is some evidence that the Leish-111f vaccine can also induce partial protection against visceral leishmaniasis in animal models (Coler RN, Goto Y, Bogatzki L, Raman V, Reed SG. Leish-111f, a recombinant polyprotein vaccine that protects against visceral Leishmaniasis by elicitation of CD4+ T cells. Infect Immun 2007;75:4648-54.), however, Leish-111f failed to protect dogs against infection and did not prevent disease development in a recent Phase III trial in dogs (Gradoni L, Foglia Manzillo V, Pagano A, Piantedosi D, De Luna R, Gramiccia M, et al. Failure of a multi-subunit recombinant leishmanial vaccine (MML) to protect dogs from Leishmania infantum infection and to prevent disease progression in infected animals. Vaccine 2005;23:5245-51.). A slightly improved version of the original construct, Leish-110f, has also been tested in dogs as a therapeutic vaccine in combination with chemotherapy and led to reduced numbers of death and higher survival probability (Miret J, Nascimento E, Sampaio W, Franca JC, Fujiwara RT, Vale A, et al. Evaluation of an immunochemotherapeutic protocol constituted of N-methyl meglumine antimoniate (Glucantime) and the recombinant Leish-110f + MPL-SE vaccine to treat canine visceral leishmaniasis. Vaccine 2008;26:1585-94.). Human Phase I and II clinical trials (safety and immunogenicity) of Leish-111f have been completed over the past few years in Brazil, Peru and Columbia, and Phase I trial has been conducted in India (http://clinicaltrials.gov).

*Leishmania* parasites are transmitted from one host to another during sand fly bite as a suspension in sand fly saliva. Sand fly saliva contains an array of molecules able to interfere with the host immune responses (Andrade BB, de Oliveira CI, Brodskyn CI, Barral A, Barral-Netto M. Role of sand fly saliva in human and experimental leishmaniasis: current insights. Scand J Immunol 2007;66:122-7.), therefore, immunity against saliva components may indirectly enhance anti-leishmanial immunity. Prior exposure of mice to bites of uninfected sand flies conferred protection from *L. major* infection (Kamhawi S, Belkaid Y, Modi G, Rowton E, Sacks D. Protection against cutaneous leishmaniasis resulting from bites of uninfected sand flies. Science 2000;290:1351-4.). Immunization with molecules present in saliva, such as maxadilan (Morris RV, Shoemaker CB, David JR, Lanzaro GC, Titus RG. Sandfly maxadilan exacerbates infection with Leishmania major and vaccinating against it protects against L. major infection. J Immunol 2001 ;167:5226-30.) or a 15 kDa protein, SP15 (Valenzuela JG, Belkaid Y, Garfield MK, Mendez S, Kamhawi S, Rowton ED, et al. Toward a defined anti-Leishmania vaccine targeting vector antigens: characterization of a protective salivary protein. J Exp Med 2001;194:331-42.), also induced protection against cutaneous leishmaniasis. More recently, it has been shown that vector salivary proteins, in particular LJM19, protect hamster from VL, (Gomes R, Teixeira C, Teixeira MJ, Oliveira F, Menezes MJ, Silva C, et al. Immunity to a salivary protein of a sand fly vector protects against the fatal outcome of visceral leishmaniasis in a hamster model. Proc Natl Acad Sci USA 2008;105:7845-50.) and immunization of dogs with salivary antigens led to the development of high IgG2 antibody levels and significant IFN-γ production (Collin N, Gomes R, Teixeira C, Cheng L, Laughinghouse A, Ward JM, et al. Sand fly salivary proteins induce strong cellular immunity in a natural reservoir of visceral leishmaniasis with adverse consequences for Leishmania. PLoS Pathog 2009;5:e1000441.).

These scientific findings make clear that vaccine development for the prevention or treatment of leishmaniasis infections are a challenging task. It becomes even more difficult in light of the different *Leishmania* subspecies that cause different forms of the disease such as cutaneous leishmaniasis (CL), mucocutaneous leishmaniasis (ML) or the severe form of visceral leishmaniasis (VL).

Against this background, it is object of the present invention to provide alternative protein or polypeptide based vaccines which are efficient in the treatment of prevention of different forms of leishmaniasis in particular for the prevention or treatment of cutaneous leishmaniasis (CL).

This problem is solved by a vaccine comprising a polypeptide or protein selected from the group consisting of p54, Q4Q8Z6, Q4Q6L9, Q4QHT2, E9AE98, or an immunologically effective fragment thereof. Preferred embodiments are part of the sub-claims.

The inventors of the present invention identified specific polypeptides and proteins isolated from *L. major* lysates that serve as efficient vaccines for the prevention or treatment of leishmaniasis. In more particular, the immunogenic protozoan-specific proteins and polypeptide of the present invention were identified by mass spectrometry and their ability to induce a strong Th1/Tc1 cell-associated cytokine profile *in vitro* upon restimulation of primed C57BL/6 lymph node cells. A variety of proteins or polypeptide could be identified using this approach and tested regarding their capacity to induce immune tolerance against *Leishmania* infection. The results of the invention show that among the variety of proteins and peptides, four candidates, named as p54, Q4Q8Z6, Q4Q6L9, E9AE98, could be isolated that were able to protect resistant C57BL/6 and susceptible BALB/c mice (only proteins Q4Q8Z6, Q4Q6L9, E9AE98) mice against leishmaniasis challenge as compared to control mice. Lesion volumes were significantly reduced in mice immunized with p54, Q4Q8Z6, Q4Q6L9 or E9AE98.

It is apparent to the person skilled in the art that an addition, omission or substitution of one or more amino acids in these proteins or polypeptide does not necessarily affect the capacity of the herein identified polypeptide or proteins to be suitable as vaccine or as medicament for a therapeutic leishmaniasis treatment. Therefore, the invention also covers truncated, elongated or modified forms of p54, Q4Q8Z6, Q4Q6L9, Q4QHT2 and/or E9AE98, and immunologically effective fragments thereof. The amino acids or nucleic acids of p54, Q4Q8Z6, Q4Q6L9, Q4QHT2 or E9AE98 may also be part of a larger protein, antibody or artificial molecule exhibiting the same pharmaceutical activity as the native forms of p54, Q4Q8Z6, Q4Q6L9, Q4QHT2 or E9AE98.

The term 'immunologically effective fragment' relates to a protein or polypeptide that has the same biological function and activity as the native p54, Q4Q8Z6, Q4Q6L9, Q4QHT2 or E9AE98 protein or polypeptide. The biological activity to serve as a vaccine can be measured, for instance, by a reduction of ear lesions in mice infected with *L. major.* However, also other methods or systems allowing determining the suitability of an agent to serve as a vaccine against leishmaniasis fall under the scope of said definition. The term for example comprises extended or truncated versions of p54, Q4Q8Z6, Q4Q6L9, Q4QHT2 or E9AE98 proteins or polypeptide, which is reflected by a reduced or increased number of amino acids in the respective amino acid sequence or nucleic acids in the respective nucleic acid sequence as compared to the p54, Q4Q8Z6, Q4Q6L9, Q4QHT2 or E9AE98 proteins or polypeptide defined in SEQ ID NO: 1 to 5.

The invention also covers homologous proteins of *L. major* p54, Q4Q8Z6, Q4Q6L9, Q4QHT2 or E9AE98 in other *Leishmania* subspecies.

The p54 peptide is a MHC class I-restricted polypeptide comprising 9 amino acids and is part of a house-keeping protein which can be found in different *Leishmania* subspecies. The amino acid sequence of p54 is identified in SEQ ID NO: 1. In a preferred embodiment, the p54 of the present invention has at least 90% sequence identity, preferably 100 % sequence identity to a polypeptide comprising an amino acid sequence as set forth in SEQ ID NO: 1.

The Q4Q8Z6 protein has a molecular weight of 89.533 kDa and comprises an amino acid sequence having at least 70% sequence identity to a polypeptide or protein comprising an amino acid sequence as set forth in SEQ ID NO: 2. In an even more preferred embodiment Q4Q8Z6 comprises an amino acid sequence comprising at least 80%, preferably at least 90% sequence identity to a polypeptide or protein comprising and amino acid sequence as set forth in SEQ ID NO: 2.

The Q4Q6L9 protein has a molecular weight of 80.009 kDa and comprises an amino acid sequence having at least 70% sequence identity to a polypeptide or protein comprising an amino acid sequence as set forth in SEQ ID NO: 3. In an even more preferred embodiment, Q4Q6L9 comprises an amino acid sequence at least 80%, preferably at least 90% sequence identity to a polypeptide or protein comprising an amino acid sequence as set forth in SEQ ID NO: 3.

The Q4QHT2 protein has a molecular weight of 16.825 kDa and comprises an amino acid sequence having at least 70% sequence identity to a polypeptide or protein, comprising an amino acid sequence as set forth in SEQ ID NO: 4. In an even more preferred embodiment, Q4QHT2 comprises an amino acid sequence having at least 80%, or preferably at least 90% sequence identity to a polypeptide or protein comprising an amino acid sequence as set forth in SEQ ID NO: 4.

The E9AE98 protein has a molecular weight of 49.673 kDa and comprises an amino acid sequence having at least 70% sequence identity to a polypeptide or protein comprising an amino acid sequence as set forth in SEQ ID NO: 5. In an even more preferred embodiment, E9AE98 comprises amino acid sequence having at least 80%, preferably at least 90% sequence identity to a polypeptide or protein comprising an amino acid sequence as set forth in SEQ ID NO: 5.

The vaccines of the person invention have been identified as being biologically effective agents that provide a protective capacity against an infection mediated by *Leishmania* pathogens. The inventors provide both *in vitro* and *in vivo* data that exemplify the protective effect against and their potential as vaccine against *Leishmania* infection, in particular a *Leishmania* infection that results in cutaneous leishmaniasis. As all the data raised in mice are also indicative for the situation in humans and other mammalian animals, it is apparent that the effect of the vaccines of the present can be generalized. Mice treated with the vaccines of the present invention showed a significant decrease of severe lesions mediated by *Leishmania* infection, indicating that p54, Q4Q8Z6, Q4Q6L9 and E9AE98 isolated from *L. major* lysates and their homologs from other *Leishmania* species are immunologically effective agents for the treatment of leishmaniasis, in particular cutaneous leishmaniasis. In particular, the vaccines of the present invention promote a protection in mice after infection with *Leishmania* and also protected susceptible BALB/c mice from progressive disease. Protection against infection was accompanied by significantly smaller ear lesion development as compared to mice treated without vaccine. Furthermore, the vaccines of the present invention resulted in lower local and systemic parasite loads in C57BL/6 mice post-infection as compared to infected control mice. CD4⁺ T cells were identified as being primarily responsible for vaccination efficiency. As a control, depletion of CD4⁺ T cells by intraperitoneal injection of anti-CD4 antibodies during immunization of C57BL/6 mice led to a loss of protection following infection. The data disclosed herein indicate that the newly identified vaccines of the present invention are effective protective agents for the prevention of the development of leishmaniasis both in the human and animal body.

The present invention not only covers a protein or polypeptide comprising one of the amino acid sequences as set forth in SEQ ID NO: 1, SEQ ID NO: 2, SEQ ID NO: 3, SEQ ID NO: 4 or SEQ ID NO: 5, or an immunologically effective fragment thereof but also a vaccine composition that contains one or more polypeptide or protein selected from the group consisting of p54, Q4Q8Z6, Q4Q6L9, Q4QHT2 or E9AE98, or an immunologically effective fragment thereof, or a homolog in any other *Leishmania* species.

Homologs of *L. major* p54, Q4Q8Z6, Q4Q6L9, Q4QHT2 or E9AE98 sharing a high degree of similarity or identity with a homologous species in *L.tropica, L. major, L. aethiopica, L. infantum, L. donovani, L. mexicana, L. venezuelensis, L. viannia, L. amazonensis, L. donovani* or a subgenus thereof, fall also within the scope of the invention. Homologous proteins/polypeptides to p54, Q4Q8Z6, Q4Q6L9 or E9AE98 can also be found in lysates from other *Leishmania* subspecies.

The invention also covers vaccines which are part of a gene expression vector that contains of p54, Q4Q8Z6, Q4Q6L9, Q4QHT2, E9AE98, or a combination thereof. In a preferred embodiment, the vector contains immunologically effective fragments of p54, Q4Q8Z6, Q4Q6L9, Q4QHT2 or E9AE98, wherein the immunologically effective fragment exhibits the same protective or therapeutic activity as the p54, Q4Q8Z6, Q4Q6L9, Q4QHT2, E9AE98 polypeptide or protein isolated from *L. major* lysates. In a preferred embodiment, the expression vector contains a polynucleotide sequence encoding a polypeptide having at least 70%, preferably at least 80%, more preferably at least 90% sequence identity to a polypeptide comprising the amino acid sequence as set forth in SEQ ID NO: 1, SEQ ID NO: 2, SEQ ID NO: 3, SEQ ID NO: 4, SEQ ID NO: 5. It is apparent to the person skilled in the art, that also truncated or prolonged forms of p54, Q4Q8Z6, Q4Q6L9, Q4QHT2, E9AE98 may have the same activity as a vaccine as the protein or polypeptide described herein. Therefore, the present invention also covers proteins and polypeptides that comprise any of the sequences as set forth in SEQ ID NO: 1, SEQ ID NO: 2, SEQ ID NO: 3, SEQ ID NO: 4 or SEQ ID NO: 5.

The present invention also relates to a pharmaceutical composition comprising a vaccine according to the present invention, and a pharmaceutically acceptable vehicle, diluent, adjuvant or excipient for use as a medicament. The present invention also covers a pharmaceutical composition comprising a vaccine of the present invention, and a pharmaceutically acceptable vehicle, diluent, adjuvant or excipient in the treatment or prevention of leishmaniasis in humans or animals. In a preferred embodiment, p54, Q4Q8Z6, Q4Q6L9, Q4QHT2 and/or E9AE98 isolated from *L. major* lysates or a homolog thereof from another *Leishmania* subspecies is used in the treatment or prevention of cutaneous leishmaniasis in humans or animals.

The *in vitro* and *in vivo* data generated by the inventors show a protective and therapeutic effect in the prevention or treatment of cutaneous leishmaniasis caused by *L. major.* It is apparent to a person skilled in the art that also other *Leishmania* subspecies such as *L. infantum* are covered by the present invention, which is due to the co-existence of different *Leishmania* subspecies in endemic areas. Therefore, the cross-protective effects against infections with different subspecies are of high interest for the vaccine development. As exemplified herein, PBMCs from two *L. infantum-infected* patients showed enhanced proliferation after *ex vivo* restimulation with the recombinant proteins of the present invention Q4Q8Z6, Q4Q6L9, Q4QHT2, E9AE98 as compared to PBMCs from healthy donors. Therefore, the pharmaceutical compositions containing a vaccine according to the present invention are suitable for the prevention or treatment of cutaneous, mucocutaneous or visceral leishmaniasis.

Preferably, the vaccine of the present invention is effective against infections mediated by *L. tropica, L. major, L. aethiopica, L. infantum, L. donovani, L. mexicana, L. venezuelensis*, or a sub-genus thereof. Infections caused by these *Leishmania* species result in the clinical picture of cutaneous leishmaniasis. The present invention also covers p54, Q4Q8Z6, Q4Q6L9, Q4QHT2, E9AE98, or an immunologically effective fragment thereof in the prevention or treatment of mucocutaneous leishmaniasis that is caused by infection with *L. viannia* or *L*. *amazonensis.* The present invention also relates to a vaccine selected from the group covering p54, Q4Q8Z6, Q4Q6L9, Q4QHT2, E9AE98, or an immunologically effective fragment thereof for the treatment or prevention of visceral leishmaniasis that is caused by infection with *L. donovani* or *L. infantum.*

In summary, the vaccines of the present invention as identified herein and characterized by p54 comprising an amino acid sequence as set forth in SEQ ID NO: 1, Q4Q8Z6 as comprising an amino acid sequence as set forth in SEQ ID NO: 2, Q4Q6L9 as comprising an amino acid sequence as set forth in SEQ ID NO: 3, Q4QHT2 as comprising an amino acid sequence as set forth in SEQ ID NO: 4, E9AE98 as comprising an amino acid sequence as set forth in SEQ ID NO: 5, or an immunologically effective fragment thereof, or their homologs in other *Leishmania* subspecies are suitable in the prevention or treatment of different forms of leishmaniasis, in particular for the treatment of the mostly occurring form of cutaneous leishmaniasis. As such, the polypeptide and proteins of the invention are suitable as vaccines in humans or mammalian animals such as the treatment of canine, equine, or feline cutaneous leishmaniasis.

The invention is illustrated in more detail in the following examples.

### Results for p54:

To expedite the generation of a vaccine, the inventors identified the most abundant proteins expressed by both life-forms - infectious-stage promastigotes and intracellular amastigotes - by mass spectrometry. Based on their predicted immunoreactivity using a computer-based algorithm (SYFPEITHI), the inventors next choose 300 H2-D^{b} and H2-K^{b} peptides derived from both *Leishmania* life-forms for further analysis. Additionally, all selected peptides were analyzed for their binding-stability towards MHC class-I molecules. A correlation between the predicted affinity and measured stability of the peptides towards MHC class-I molecules was not observed. Further, all 300 peptides were tested in *in vitro* experiments. Peptides were co-cultured with C57BL/6 DC and primed CD8⁺ T cells for 48 hrs. Supernatants were assayed for the amounts of secreted IFNγ, IL-17A, IL-4 and IL-10 by ELISA. Based on their cytokine-profile, the inventors subsequently selected 22 peptides for evaluation of their potential to protect C57BL/6 mice against challenge with *L. major.* Mice were immunized in a prime/boost/boost approach with 20 µg peptide followed by 2x 10 µg peptide in combination with CpG as adjuvant in one ear. One week later, the mice were infected with physiological low-dose inocula of 1,000 metacyclic promastigotes in the contralateral ear and lesion volumes were measured weekly. Interestingly, one peptide (p54) was able to protect mice against challenge compared to control mice, whereas all other tested peptides failed. Lesion volumes were significantly reduced in p54-immunized mice compared to control mice. Additionally, local and systemic parasite burdens were analyzed. In line with reduced lesion volumes, mice immunized with p54 showed reduced numbers of parasites in their ears and spleens.

### Materials and Methods for p54

### Mice

Female C57BL/6J mice - 6-8 weeks old - were purchased from Janvier Labs (France). Animals were housed under specific pathogen free (SPF) conditions in the Translational Animal Research Center (TARC) of the Johannes Gutenberg-University, Mainz, Germany and in accordance with institutional and federal guidelines. All experiments were undertaken with approved license from the Animal Care and Use Committee of the Region Rhineland-Palatinate, Germany.

### Mass spectrometry, binding affinity and binding stability

The most abundant proteins of infectious-stage promastigotes as well as intracellular amastigotes were identified by mass spectrometry. Using a computer-based Algorithm - SYFPEITHI - all 8-mer and 9-mer peptides derived from these proteins were evaluated for their predicted affinity to bind to C57BL/6-specific MHC I-molecules as described before (Dietze-Schwonberg K, Grewe B, Brosch S, Kuharev J, van Zandbergen G, Rammensee HG, Tenzer S and von Stebut E. In silico prediction of Leishmania major-specific CD8+ epitopes. Exp Dermatol, 2017, pii: S0923-1811(17)30761-2. doi: 10.1016/j.jdermsci.2017.06.014). A high SYFPEITHI score indicates a high binding affinity of the peptide towards MHC molecules.

Finally, 300 selected peptides were analyzed for their binding stability towards MHC I-molecules -as described previously (Harndahl, M., Rasmussen, M., Roder, G., Buus, S. Real-time, high-throughput measurements of peptide-MHC-I dissociation using a scintillation proximity assay. J of Immunol Meth, 2011, 374: 5-12.).

### Generation and co-culture of DC with CD8⁺ T cells

DC were generated from bone marrow of C57BL/6 mice using GM-CSF and IL-4. Immature DC were harvested on day 6, plated at 2x10⁵ cells/mL in RPMI (Biochrome) with 5% FCS and stimulated overnight with LPS (100 ng/mL). T cell subsets were enriched from draining lymph nodes of infected (wk 3-4) C57BL/6 mice using anti-CD8⁺ microbeads (α-mouse Ly-2, Miltenyi). Stimulated DC were irradiated for 4 min at 55 kV, 40 Gy and then plated at 1x10⁵ cells/200 µL in RPMI (Biochrome) with 5% FCS. The cell viability was microscopically assessed by trypan blue (Sigma-Aldrich) exclusion. DC were co-cultured with T cells (DC/T-cell ratio: 1:5) and different peptides (1x10⁻⁶mol) for 48 hrs. at 37°C in 5% CO₂. Afterwards, the supernatant was used for determination of secreted IFNγ, IL-17A, II-4 and IL-10 using ELISA - according to manufacturer's guidelines (R&D for IFNγ and IL-17A/BD for IL-4 and IL-10).

### Antibodies and flow cytometry

All monoclonal antibodies used for T cell quantification were purchased from BD Pharmingen (Heidelberg, Germany). In order to analyze purity of CD8⁺ T cells after enrichment with CD8 microbeads, cells were stained with PE-labeled α-mouse CD3ε (clone: 145-2C11) and FITC-labeled α-mouse CD8a (Ly-2, clone: 53-6.7). In addition, purified cells were stained with antibodies against PE-labeled α-mouse CD3ε and FITC-labeled α-mouse CD4 (L3T4, clone: RM4-5) to analyze contamination with CD4⁺ T cells.

Six days after isolation from bone-marrow, DC were harvested and purity of DC was assessed. Monoclonal antibodies were purchased from BD Pharmingen, Serotec or eBioscience. To quantify purity of DC, all cells were stained with PE-labeled CD11c (clone N418 | eBioscience) and FITC-labeled α-mouse MHC-I (H-2D^{b}, clone: 2G5 | Serotec), α-mouse MHC-II (I-A/I-E, clone: 2G9 | BD Pharmingen) or α-mouse CD86 (B.7-2, clone: GL1 | BD Pharmingen) respectively. For intracellular FACS-staining, LN cells of immunized and infected C57BL/6 mice were isolated and plated at 10x10⁶ cells/mL in RPMI (Biochrome) with 5% FCS. All cells were stimulated with Brefeldin A (1 µg/mL). Some cells were restimulated with peptides (1x10⁻³mol) or with PMA (50 ng/mL), lonomycin (500 ng/mL) as positive control for 6 hrs. Cells were then stained with PE-Cy7-labeld α-mouse CD8 (clone 53-6.7 | eBioscience), α-mouse APC-Cy7-labeled CD4 (clone: GK1.5 | eBioscience) and eFluor 450-labeled α-mouse CD3ε (clone eBio500A2 | eBioscience), as well as APC-labeled α-mouse IFNγ (clone: XMG1.2 | eBioscience) and α-mouse IL-17A (clone: ebio17B7 | eBioscience). Cells were analyzed using a LSRII cytometer (BD Bioscience) and FlowJo software (Tree Star).

### Parasites and infection

Infectious-stage metacyclic promastigotes were isolated from stationary cultures of *L. major* clone V1 (MHOM/IL/80/Friedlin) by positive selection using a biphasic Ficoll gradient (10%/20%) as described previously (Von Stebut E, Ehrchen JM, Belkaid Y, Kostka SL, Molle K, Knop J, Sunderkotter C, Udey MC. Interleukin 1alpha promotes Th1 differentiation and inhibits disease progression in Leishmania major-susceptible BALB/c mice. J Exp Med, 2003, 198: 191-199). Infections were initiated intradermally in ear skin of mice using standard high dose (2x10⁵) or physiological low dose inocula with 1x10³ metacyclic promastigotes per ear. Groups of five mice per experiment were immunized intradermally in a prime/boost/boost approach with 20 µg peptide + 10 µg CpG oligodeoxynucleotides 1826 (CpG ODN 1826, InvivoGen) as adjuvant followed by two further immunizations with 10 µg peptide + 10 µg CpG ODN 1826 per ear. As negative control, mice were immunized with PBS + 10 µg CpG ODN 1826. One week later, all mice were infected with physiological relevant low-dose of 1,000 metacyclic promastigotes in a volume of 20 µL into the opposite ear. Lesion volumes were measured weekly in three dimensions and are reported as ellipsoids ((a/2 x b/2 x c/2) x 4/3 x π). Local and systemic parasite burdens were enumerated using a limiting dilution assay, as described before (Von Stebut E, Ehrchen JM, Belkaid Y, Kostka SL, Molle K, Knop J, Sunderkotter C, Udey MC. Interleukin 1alpha promotes Th1 differentiation and inhibits disease progression in Leishmania major-susceptible BALB/c mice. J Exp Med, 2003, 198: 191-199).

### Statistics

Results represent means ± SEM. Statistical analysis was determined using StatView (Cary, NC) software for Windows. The unpaired Student's *t* test was used to determine statistical significance. P values less than 0.05 were considered significant and are indicated in the corresponding figures (* *p* ≤ 0.05; ** *p* ≤ 0.005; *** *p* ≤ 0.002).

### Results for Q4Q8Z6, Q4Q6L9, Q4QHT2, E9AE98:

Soluble Q4Q8Z6, Q4Q6L9, Q4QHT2, E9AE98_proteins were isolated from highly immunogenic *L. major* lysate and subsequently fractionated by two-step anion exchange chromatography.

Several eluted fractions induced a strong Th1/Tc1 cell-associated cytokine profile *in vitro* upon restimulation of primed C57BL/6 lymph node cells. Within these reactive fractions the inventors identified 36 *L*. *major*-specific proteins by mass spectrometry, of which 4 proteins (90/80/17/50 kDa) were recombinantly expressed in *E. coli.* Interestingly, immunization studies *in vivo* with 1 µg of recombinant protein plus addition of CpG as adjuvant revealed that only the 80 kDa protein (Q4Q8Z6) significantly promoted protection in C57BL/6 mice and susceptible BALB/c after infection with live *L. major* promastigotes. In addition, the 50 kDa protein (E9AE98) also protected BALB/c mice from progressive disease. Protection against infection was accompanied by significantly smaller ear lesion development compared to mice treated with CpG alone, and by lower local and systemic parasite loads in C57BL/6 mice post-infection compared to infected control mice.

CD4⁺ T cells were identified as T cell subset primarily responsible for vaccination efficacy. Depletion of CD4 cells by intraperitoneal injection of anti-CD4 antibodies during immunization of C57BL/6 mice led to a loss of protection after infection. In contrast, infected mice lacking CD8⁺ T cells during immunization were still protected. Finally, the inventors determined the restimulating capacity of the 4 proteins on human peripheral blood mononuclear cells (PBMCs) *ex vivo* from three patients with prior *L. major* infection. As determined by incorporation of (³H)-TdR by PBMCs after incubation with recombinant protein (5-20 µg/ml) for 5 d, all 4 single proteins induced proliferation of PBMCs comparable to those after incubation with total *L. major* lysate, while healthy controls PBMCs did not proliferate. Due to the co-existence of different *Leishmania* subspecies in endemic areas, the cross-protective effect against infections with different subspecies is apparent and thus of high interest for the vaccine development.

Sequence alignment analysis (BLAST) revealed 87-97% amino acid sequence identity of the *L*. *major* protein candidates Q4Q8Z6, Q4Q6L9, Q4QHT2,and E9AE98 of the invention with the corresponding *L. infantum* proteins. PBMCs from two *L. infantum-infected* patients showed enhanced proliferation after *ex vivo* restimulation with the 4 recombinant proteins compared to PBMCs from healthy donors. With regard to a favored cross-protective effect, the newly identified proteins were identified as new vaccine candidates against different forms of leishmaniasis and a source for protective T cell epitopes.

### Materials and Methods for Q4Q8Z6, Q4Q6L9, Q4QHT2, E9AE98

### Mice

Female C57BL/6J and BALB/cJ mice, 6-8 weeks old, were purchased from Janvier Labs (France). Animal experimentation was conducted in accordance with relevant laws, permission of the Ethics Committee of the state Rhineland-Palatinate, Germany, current institutional guidelines and the Helsinki convention for the use and care of animals.

### Parasites and antigen preparations

Infectious stage metacyclic promastigotes of *L. major* clone V1 (MHOM/IL/80/Friedlin) were isolated from stationary cultures by positive selection using a biphasic (10%/20%) Ficoll gradient as described previously (Kautz-Neu K, Kostka SL, Dinges S, Iwakura Y, Udey MC, and von Stebut E. A Role for Leukocyte-Derived IL-1RA in DC Homeostasis Revealed by Increased Susceptibility of IL-1 RA-Deficient Mice to Cutaneous Leishmaniasis. J Invest Dermatol. 2011;131(8):1650-9.). Isolated promastigotes were used for *in vivo* infection of mice and preparation of immunogenic soluble *Leishmania* antigen (SLA). SLA (consisting of 9.9 x 10¹⁰ metacyclic promastigotes in Tris buffer (10 mM Tris, pH 8 (Carl Roth)) was prepared by 10-12 freeze-thaw cycles of metacyclic promastigotes by alternating incubation in liquid nitrogen and at 37°C to avoid protein degradation, followed by subjection to sonication (Ultrasonic Rod Sonoplus HD 2070, BANDELIN electronic) on ice with 4 30-sec blasts with 50% intensity, 24x 0.5 cycles each with 1-min break interval. The lysate was microscopically viewed to ensure parasite disruption. After fill-up with Tris buffer to a final volume of 25-30 ml, SLA was sterile-filtered. For further partition, SLA was centrifuged at 3,000 x *g* for 20 min to pelletize nuclei, at 15,000 x *g* for 60 min to pelletize mitochondria. Supernatants were collected and re-centrifuged at 100,000 x *g* for 2 hrs. using an ultracentrifuge (Optima L-80 XP, Beckman Coulter). Membrane proteins were pelletized within this final step and soluble proteins (SP) revealed in the supernatants. Isolated parasitic components were resuspended in an appropriate volume of Tris buffer. SLA and all antigen preparations including sterile-filtered SP were stored at -80°C.

### Recombinant proteins

4 chosen proteins were subjected to recombinant expression in *E. coli* expression systems. The recombinant proteins Q4Q8Z6, Q4H6L9 and E9AE98 were expressed by Trenzyme, and Q4QHT2 was kindly provided by Danielle Arnold-Schild (Institute of Immunology, University Medical Center, Mainz, Germany).

### Immunization, T cell depletion and infection of mice

Groups of three to five mice per experiment were injected i.d. into one ear with different amounts of SP (0.2, 2 or 20 µg) or recombinant proteins (0.02, 0.2, 2 or 20 µg) each combined with 10 µg CpG oligodeoxynucleotides 1826 (CpG ODN 1826, InvivoGen) as adjuvant. One and three weeks later, mice were immunized i.d. with half the amount of protein, i.e. SP (0.1, 1 or 10 µg) or recombinant proteins (0.01, 0.1, 1 or 10 µg) each plus 10 µg CpG. Control mice were treated at each point in time with CpG alone. All mice were challenged one week after the final immunization with the physiologically relevant dose of 10³ live metacyclic promastigotes in a volume of 20 µl into the contralateral ear applying needles. Lesion volumes were measured weekly in three dimensions and are reported as ellipsoids ((a/2 x b/2 x c/2) x 4/3 x π).
To investigate the relevance of single T cell subtypes on protective immunization against *L. major* infection *in vivo,* CD4⁺, CD8⁺ or both CD4⁺/CD8⁺ T cells, respectively, were depleted during the immunization period of mice. Neutralizing monoclonal antibodies (anti-CD4 (GK1.5) mAb, 125 µg/mouse; anti-CD8 (YTS169) mAb, 50 µg/mouse; Bioceros) were diluted in PBS and injected i.p. in a final volume of 100 µl 24 hrs. before each immunization. Control mice received only PBS by i.p. injection. After 3-4 weeks post-immunization, i.d. infection with 10³ *L. major* promastigotes was initiated after recurrence of at least 60% of the T cell compartment.

### LN cell preparation and cytokine profiles

The submandibular ear skin draining LNs were isolated from immunized and/or *L. major* infected C57BL/6 mice and single cell suspensions prepared by mechanical dissociation. The cell viability was microscopically assessed by trypan blue (Sigma-Aldrich) exclusion. 1x10⁶ LN cells were dispended into 96-well plates (BD Pharmingen), cultured in 200 µl complete RPMI 1640 medium (Biochrome) without antigen (Ø) or in the presence of titrated SLA fractions (10 µl each), SLA (50 µg/ml), SP (50 µg/ml), OVA (50 or 100 µg/ml) or recombinant *L*. *major*-specific protein (10 or 50 µg/ml). Cultures were incubated at 37°C in 5% CO₂. 48 hrs. supernatant fluids were harvested and analyzed for the amounts of secreted IFNγ, IL-4 and IL-10 by ELISA according to the manufacturer's instructions.

### Antibodies and flow cytometry

All monoclonal antibodies used for T cell quantification were purchased from eBioscience. In order to quantify cytotoxicity rates, draining LN cells from C57BL/6 mice with healed *L. major-*infection were analyzed 48 hrs. after restimulation using phycoerythrin (PE)-labeled anti-mouse CD3ε (clone eBio500A2) antibody or the corresponding isotype PE-labeled golden syrian hamster IgG, respectively, and stained with propidium iodide (PI; 1 µg/ml) shortly before measurement. To assess cell proliferation *in vitro,* draining LN cells from *L*. *major*-infected, healed C57BL/6 mice were labeled using the CFDA-SE Cell Tracer Kit (Invitrogen) in accordance to the manufacturer's instructions. CFSE-labeled LN cells (1 µM) were antigen-specifically restimulated with the stimulants mentioned above and incubated at 37°C, 5% CO₂. After 5 d, single cell suspensions were stained for surface markers using the following monoclonal antibodies: eFluor 450-labeled anti-mouse CD3ε (clone eBio500A2), allophycocyanin (APC)-labeled anti-mouse CD4 (clone GK1.5) and phycoerythrin-cyanine (PE-Cy7)-labeld anti-mouse CD8 (clone 53-6.7). The following corresponding isotype controls (eBioscience) were used: eFluor 450-labeled golden syrian hamster IgG, APC-labeled rat IgG2b, κ and PE-Cy7-labeled IgG2a, κ. Cells were analyzed using a LSRII cytometer (BD Bioscience) and FlowJo software (Tree Star).

### Anion exchange chromatography

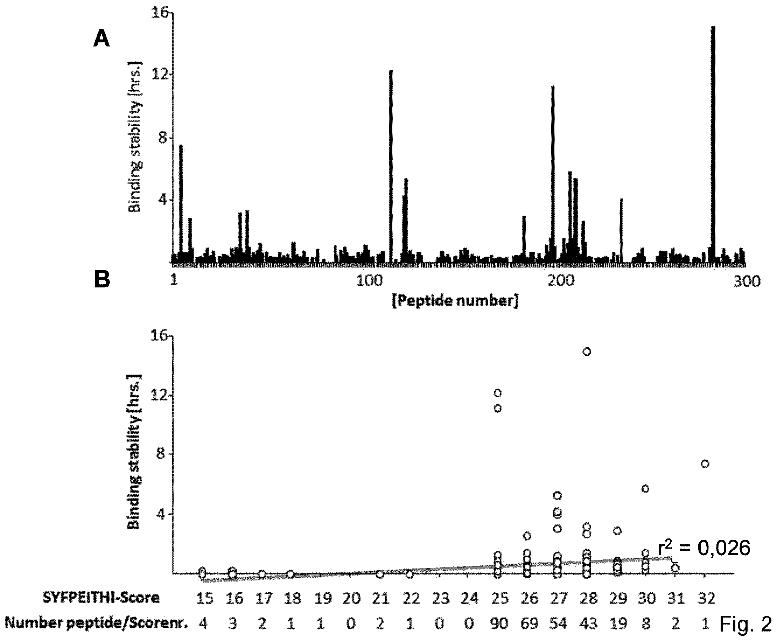

Two-step anion exchange chromatographic fractionation was performed using an ÄKTA purifier 10 (GE Healthcare). 22 ml sterile-filtered SP solution were applied to an HR-16/50 column (GE Healthcare) pre-packed with diethyl aminoethyl (DEAE) cellulose (DEAE-650S Toyopearls; Tosoh Bioscience) at a flow rate of 3 ml/min in 10 mM Tris, pH 8 (Tris buffer; Carl Roth). Proteins were eluted with a gradient of 25-50% 1 M NaCl over 100 min at a flow rate of 3 ml/min and a temperature of 4°C. Protein absorption was monitored at 280 nm. Afterwards, the column was rinsed with 100% 1 M NaCl and re-equilibrated resulting in an analysis time of 130 min in total. 4 chosen fractions pools (see Fig. 2B) were separately dialyzed against Tris buffer over night at 4°C to remove co-eluated salt ions using dialysis tubings with 6-8 kDa size exclusion (ZelluTrans/Carl Roth). Dialyzed fraction pools I-IV were successively applied to a MonoQ 5/50 GL column (GE Healthcare) equilibrated overnight at 4°C with Tris buffer and a flow rate of 0.02 ml/min. For protein elution, a linear gradient was applied up to 75% 1 M NaCl over 150 min per fraction pool followed by 10 min with 100% 1 M NaCl at a flow rate of 0.5 ml/min. The column re-equilibration was performed with Tris buffer over 10 min at 4°C and the same flow rate conditions as described before.

### Sample preparation and mass spectrometric analysis

Prior to tryptic digestion, fractions were lyophilized and resolubilized in lysis buffer (7 M urea, 2 M thiourea, 5 mM DTT, 2% CHAPS) by sonication for 10 min at 4°C. Protein amounts were determined using the Pierce 660 nm Protein Assay (Thermo). Proteins (20 µg) were digested applying a modified FASP protocol (Distler U, Kuharev J, Navarro P, Levin Y, Schild H, and Tenzer S. Drift time-specific collision energies enable deep-coverage data-independent acquisition proteomics. Nat Methods. 2014;11(2):167-70.). Prior to LC-MS analysis, peptides were dissolved in 0.1% formic acid and spiked with 20 fmol/µl of enolase 1 MassPREP™ protein digestion standard (Waters). Tryptic peptides were analyzed in triplicates using a nanoscale UPLC system (nanoAcquityUPLC (Waters)) coupled to a Q-TOF Premier mass spectrometer (Waters). Peptides were separated on a HSS-T3 C18 1.7 µm, 75 µm x 150 mm reversed-phase column (Waters) using direct injection mode as described before (Distler U, Kuharev J, Navarro P, Levin Y, Schild H, and Tenzer S. Drift time-specific collision energies enable deep-coverage data-independent acquisition proteomics. Nat Methods. 2014;11(2):167-70.). Water containing 0.1% formic acid was used as mobile phase A and ACN containing 0.1% formic acid as mobile phase B. Peptides were eluted with a gradient of 5-40% mobile phase B over 90 min at a flow rate of 300 nl/min and a temperature of 55°C. Afterwards, the column was rinsed with 90% mobile phase B and re-equilibrated resulting in a total analysis time of 120 min. Eluted peptides were analyzed in positive mode ESI-MS using MS^{E} as described in detail by Distler et al. (Distler U, Kuharev J, Navarro P, Levin Y, Schild H, and Tenzer S. Drift time-specific collision energies enable deep-coverage data-independent acquisition proteomics. Nat Methods. 2014;11(2):167-70.). The data were post-acquisition lock mass corrected using [Glu1]-Fibrinopeptide B.

### Protein identification by database search

LC-MS data were processed using ProteinLynxGlobalSERVER version 3.0.2 (PLGS, Waters Corporation) searching against the RefSeq *Leishmania* database, which was concatenated to a reversed decoy database. For database search, precursor and fragment ion mass tolerances were automatically determined by PLGS3.0.2. Ion mass tolerance was typically below 5 ppm (3.3 ppm RMS) for precursor and below 10 ppm for fragment ions. The following search criteria were set for peptide identification: i) trypsin as digestion enzyme ii) up to two missed cleavages allowed iii) fixed carbamidomethylcysteine and variable methionine oxidation as modifications, iv) minimum three identified fragment ions. The initial false discovery rate (FDR) for protein identification was set to 1% in PLGS.
Data were post-processed using the software package ISOQuant (Distler U, Kuharev J, Navarro P, Levin Y, Schild H, and Tenzer S. Drift time-specific collision energies enable deep-coverage data-independent acquisition proteomics. Nat Methods. 2014;11(2):167-70.) performing retention time alignment, exact-mass-retention-time clustering, signal annotation, normalization and protein isoform/homology filtering. Within ISOQuant, the peptide level FDR for cluster annotation was set to 1%. For further analysis, only proteins identified by at least two peptides (minimum length: 6 amino acids) were considered. Further on, to be included in the final dataset a protein had to be identified in all biological replicates of at least one fraction, resulting in a FDR of <1% on protein level for the complete dataset. Absolute in-sample amounts were calculated for each protein based on the TOP3 approach (Silva JC GM, Li GZ, Vissers JP, Geromanos SJ. Absolute quantification of proteins by LCMSE: a virtue of parallel MS acquisition. Molecular & Cellular Proteomics. 2006;5(1):144-56.). For display, the inventors defined the relative amount of each protein in respect to the sum over all detected proteins (ppm: parts per million (w/w) of total protein).

### Patients

Blood samples were collected from 3 donors with prior *L. major* infection and from 2 volunteers with prior *L. infantum* infection (age 43 to 68 years) for PBMC isolation and antigen-specific stimulation *ex vivo.* Blood was kept at room temperature before PBMC enrichment. PBMCs from healthy blood donors served as controls. The clinical data are summarized in Table S5.

### PBMC isolation and antigen-specific stimulation ex vivo

PBMC were isolated within 2 hrs. after blood collection and freshly used in *in vitro* experiments. PBMC isolation from heparinized blood was performed by Biocoll (Biochrom) density centrifugation. 0.5 x 10⁶ PBMCs were stimulated in triplicates in a final volume of 200 µl with tetanus toxoid (10 µg/ml; kindly provided by Helmut Jonuleit, Department of Dermatology, University Medical Center, Mainz, Germany), SLA, SP or recombinant proteins (5, 10 or 20 µg/ml each) for 5 d at 37°C, 5% CO₂. All cultures were performed in serum-free X-VIVO-15 medium (Cambrex, Verviers, Belgium). Finally, stimulated PBMCs were pulsed with (³H)-TdR (37 kBq/well; ICN, Irvine, California) for 18 hrs. and thymidine incorporation was evaluated using a liquid β-scintillation counter (1205 beta-plate; LKB Wallac, Turku, Finland). Results were displayed in mean counts per minute (cpm).

### Statistics

Results represent means ± SEM. Statistical analysis was determined using StatView (Cary, NC) or GraphPad Prism (La Jolla, CA) software for Windows. The unpaired Student's *t* test was used to determine statistical significance. For lesion volumes, the AUCs were calculated and when comparing the AUCs between 2 groups, one-way ANOVA with Bonferroni post-test was performed. P values less than 0.05 were considered significant and are indicated in the corresponding figures (* p ≤ 0.05; ** *p* ≤ 0.005; *** *p* ≤ 0.002).

### Short description of the Figures

### I. In silico

Fig. 1 Mass spectrometry revealed 551 proteins in *L. major.*
Fig. 2: Correlation between binding stability and affinity of selected peptides towards MHC I molecules

### II. In vitro

Fig. 3: DC and T cell quality FACS.
Fig. 4: Coculture of peptide-loaded DC and primed CD8⁺ T cells.
Fig. 5: Selected peptides for further *in vivo* analysis.
Fig. 6: Analysis of tested cytokines against binding affinity and stability.
Fig. 7: Analysis of tested cytokines IFNγ, IL-17A, IL-4 und IL-10.

### III. In vivo

Fig. 8: Immunization protocol: Prime/boost/boost (PBB) approach
Fig. 9: Lesion volumes after immunization with selected peptides.
Fig. 10: Number of local and systemic parasites after immunization with p54
Fig. 11: Antigen-specific cytokine release upon immunization with p54
Fig. 12: intracellular FACS-staining after immunization
Fig. 13: Soluble proteins (SP) from *L. major* lysate are immunogenic and protect against infection.
Fig.14: Reactive capacity of SP fractions *in vitro* after two-step anion exchange chromatography.
Fig. 15: Identification of *L*. *major*-specific proteins by mass spectrometry.
Fig. 16: Protein-specific induction of T cell proliferation from primed dLN.
Fig. 17: Protein-specific protective capacity against *L. major* infection *in vivo* requires CD4⁺ T cells.
Fig. 18: Antigen-specific restimulation of PBMCs from donors with prior *Leishmania* infection.

### Detailed description of the Figures

### I. In silico:

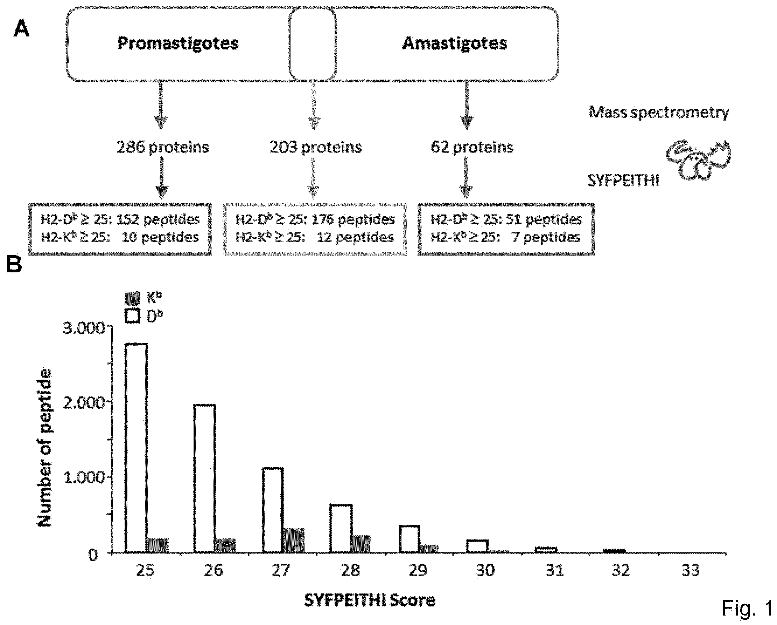

**Fig. 1** **Mass spectrometry revealed 551 proteins in *L. major.***
**A,** The most abundant proteins expressed by both life-forms of *L. major* were identified by mass spectrometry: 286 promastigote-, 62 amastigote-specific and 203 proteins common in both life-forms. Using SYFPEITHI, peptides were predicted for their affinity to bind C57BL/6-specific H2-D^{b} and H2-K^{b} MHC class I molecules. **B**, As the score is a relative indicator for binding affinity, the inventors set a cut-off of 25 for SYFPEITHI. 300 peptides that had a SYFPEITHI-score ≥ 25 were chosen for further analysis.

**Fig. 2****: Correlation between binding stability and affinity of selected peptides towards MHC I molecules. A,** In cooperation with the group of Soren Buus (University of Copenhagen, Denmark), all 300 selected peptides were analyzed for their binding stability towards MHC I molecules. **B,** A correlation between binding stability and binding affinity (SYFPEITHI Score) is not visible.

### II In vitro:

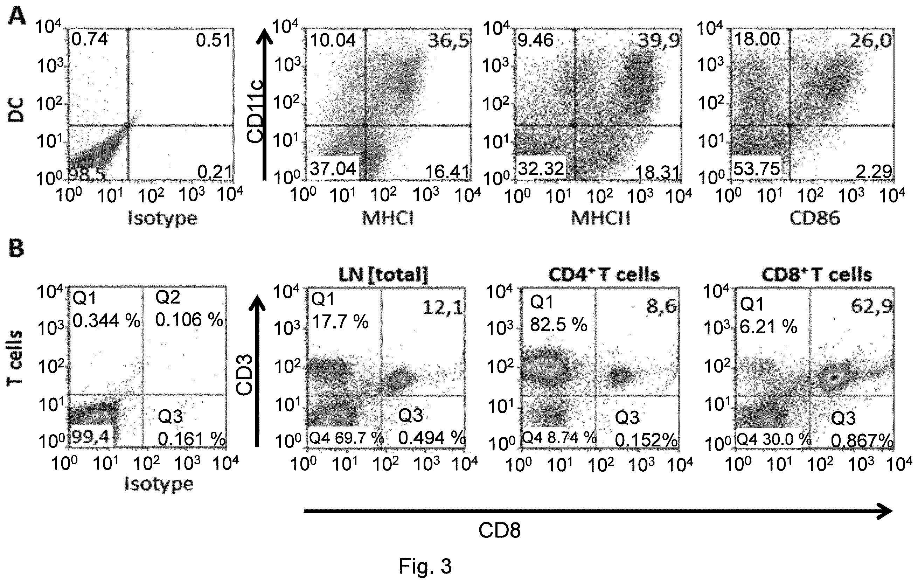

**Fig. 3****: DC and T cell quality FACS. A,** exemplarily shown, 6 days after isolation from the bone-marrow of naïve C57BL/6 mice, cells were analyzed for DC-specific marker (CD11c, MHC I, MHC II and CD86). **B,** T cells were isolated from draining lymph nodes of infected C57BL/6 mice and - exemplarily shown - analyzed specifically for CD3/CD8 double positive T cells prior as well as after MACS isolation of CD8⁺ T cells.

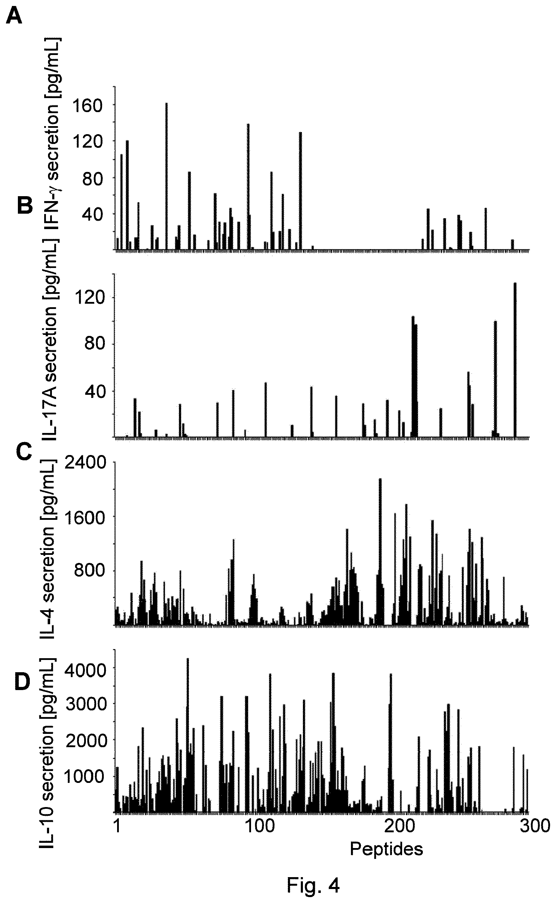

**Fig. 4****: Coculture of peptide-loaded DC and primed CD8⁺ T cells.** DC, generated from naïve C57BL/6 mice were stimulated with LPS (100 ng/mL) for 18 hrs. Afterwards, cells were irradiated (55 kV and 40 Gy). Further, DC were stimulated with 300 selected peptides and cocultured with primed, MACS-sorted CD8⁺ T cells for 48 hrs. (ratio DC:T cell = 1:5). After 2 days, supernatants were assayed for the amounts of **A,** IFNγ, **B,** IL-17A, **C,** IL-4 and **D,** IL-10 using ELISA (mean of n=4 independent experiments).

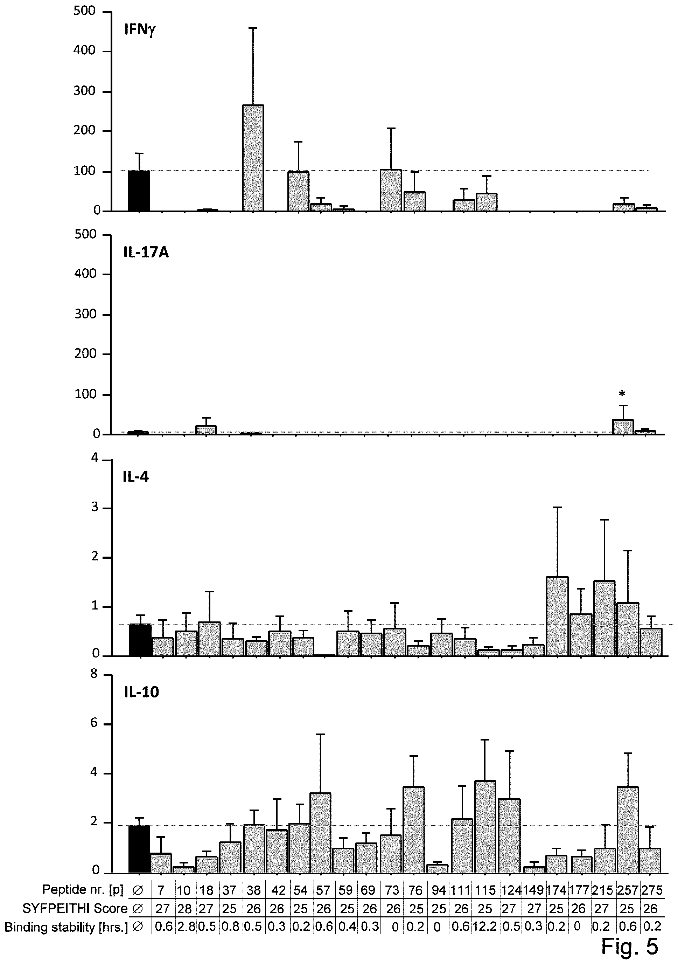

**Fig. 5****: Selected peptides for further *in vivo* analysis.** DC of naïve C57BL/6 mice were generated and cocultured with primed CD8⁺ T cells and peptides for 48 hrs. Supernatants were assayed for the amounts of secreted IFNγ, IL-17A, IL-4 and IL-10 using ELISA - exemplarily shown for the selected peptides that were tested in *in vivo* experiments (n=4, mean ± SEM, * = p≤0,05).

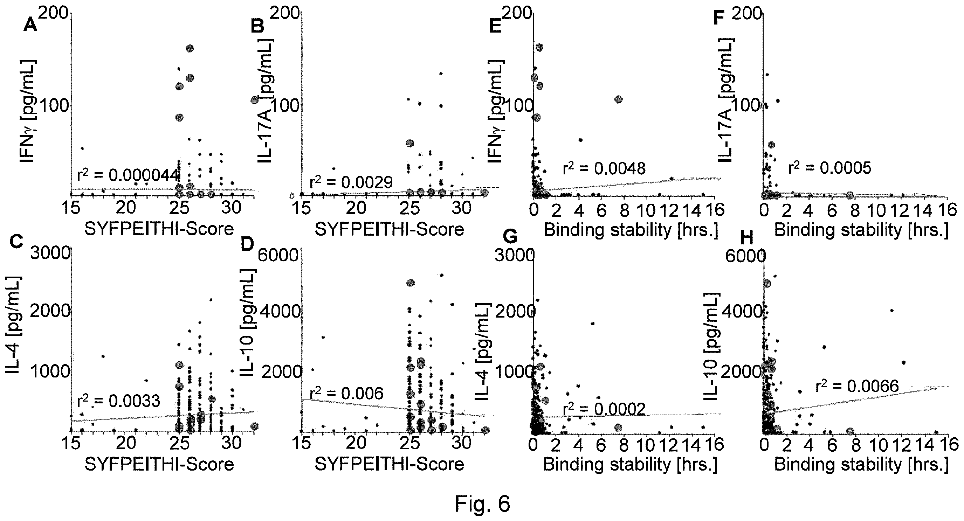

**Fig. 6****: Analysis of tested cyokines against binding affinity and stability.** All four tested cytokines - IFNγ, IL-17A, IL-4 and IL-10 - were analyzed against either binding affinity (SYFPEITHI score) or against binding stability to analyze a possible correlation. Single peptides are represented as dots (mean of n=4 independent experiments).

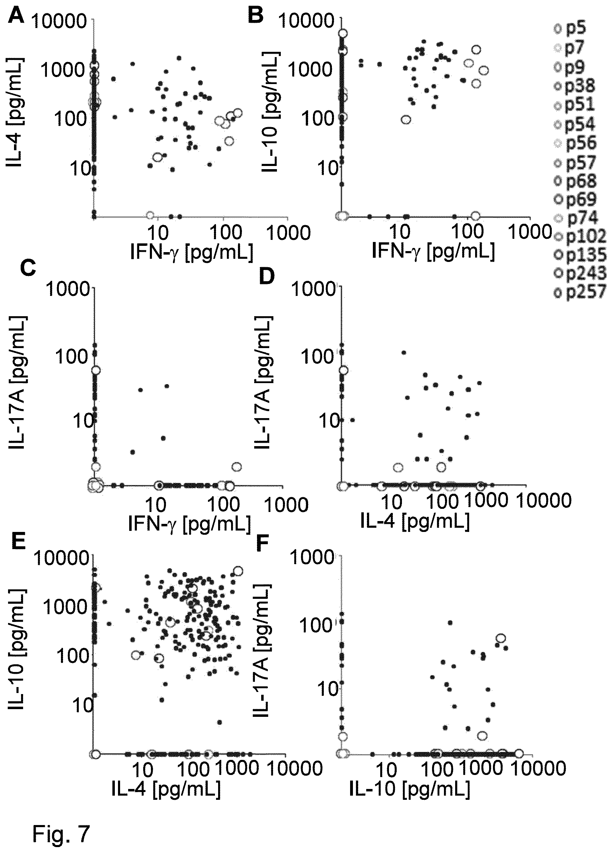

**Fig. 7****: Analysis of tested cytokines IFNγ, IL-17A, IL-4 und IL-10.** All tested cytokines were analyzed against each other to reduce the number of possible vaccine candidates (mean n=4 independent experiments).

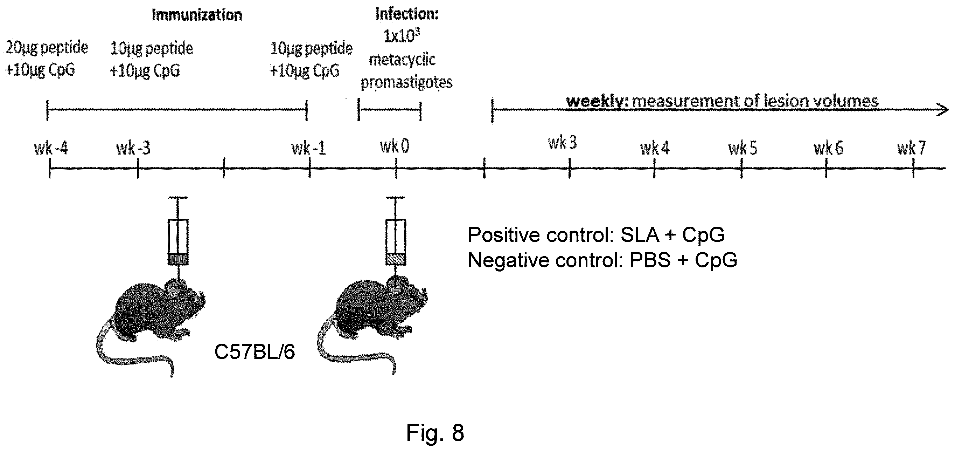

**Fig. 8****: Immunization protocol: Prime/boost/boost (P/B/B) approach.** C57BL/6 mice were immunized in a P/B/B approach with 20 µg peptide plus CpG as adjuvant followed by 2 more immunizations with 10 µg peptide in combination with CpG as adjuvant. Control mice were either immunized with soluble *Leishmania* antigen (SLA) plus CpG (positive control) or PBS plus CpG (negative control). One week later, all mice were infected with physiological relevant low dose of 1,000 metacyclic promastigotes in the alternate ear. Lesion volumes were measured weekly.

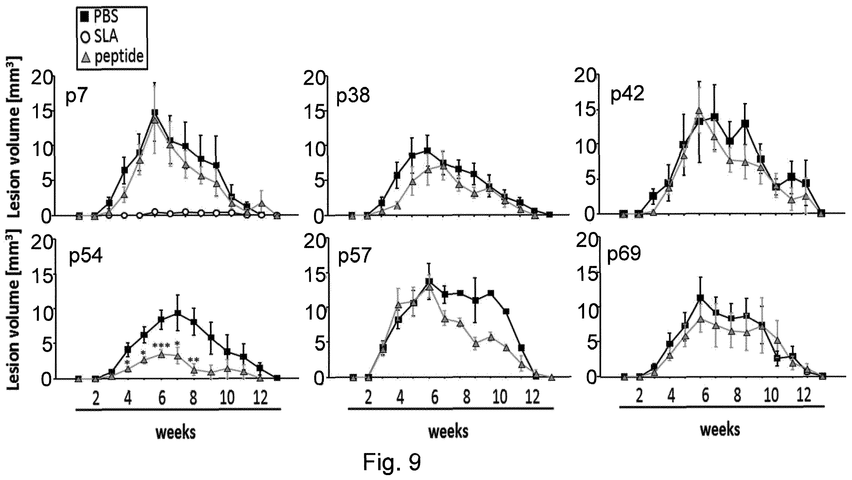

**Fig. 9****: Lesion volumes after immunization with selected peptide.** C57BL/6 mice were immunized in a P/B/B approach - exemplarily shown for 6 selected peptides. Starting with 20 µg peptide plus 10 µg CpG 1826 as adjuvant in one ear, followed by 2 more immunization steps with only 10 µg peptide + 10 µg CpG as adjuvant. One week later, the mice were infected with 1,000 metacyclic promastigotes in the contralateral ear. Lesion volumes were measured weekly and are reported as elipsoids (n=2-6 independent experiments with 8-37 mice per group; mean ± SEM; * = p≤ 0.05).

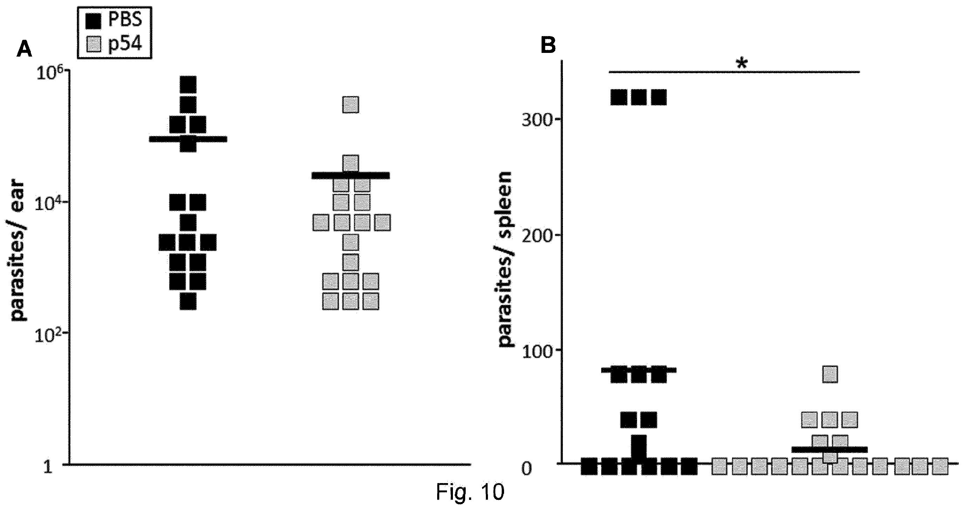

**Fig. 10****: Number of local and systemic parasites after immunization with p54.** C57BL/6 mice were immunized in a P/B/B approach. Six weeks post infection, mice were sacrificed and number of local **(A)** and systemic **(B)** parasites was analyzed using limiting dilution assay (n= 4 independent experiments with 16-19 mice per group; mean ± SEM, *p≤0.05).

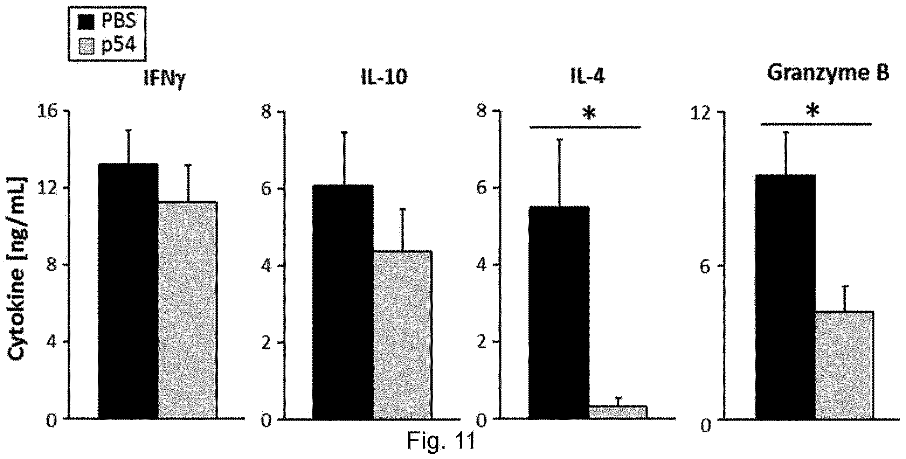

**Fig. 11****: Antigen-specific cytokine release upon immunization with p54.** Six weeks post infection LN cells of immunized (PBS or p54) C57BL/6 mice were restimulated with SLA (25 µg/mL) for 48 hrs. Supernatants were assayed for the amounts of secreted IFNγ, IL-10, IL-4 and Granzyme B (n= 2-3 independent experiments with 7-14 mice per group; mean ± SEM, *p≤0.05).

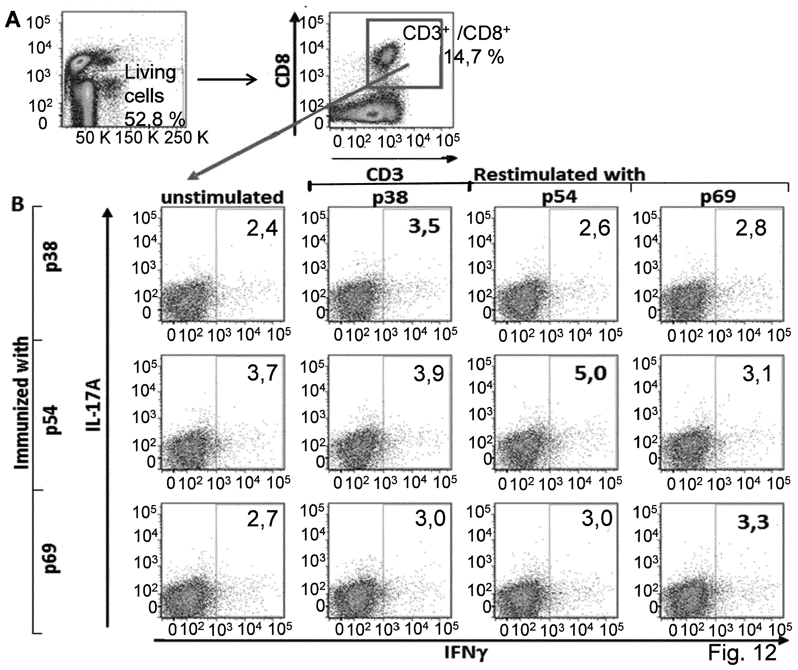

**Fig. 12****: intracellular FACS-staining after immunization.** C57BL/6 mice were immunized with 3 different peptides in a P/B/B approach. Six weeks post infection LN cells were restimulated with the same or the other 2 peptides that were prior used for immunization for 6 hrs. Cells were gated on CD3/CD8 double positives and further on IL-17A and IFNγ. Cells that were restimulated with the same peptide as the immunization showed increased levels of IFNγ producing CD3/CD8 T cells. (n=1 exemplarily shown experiment).

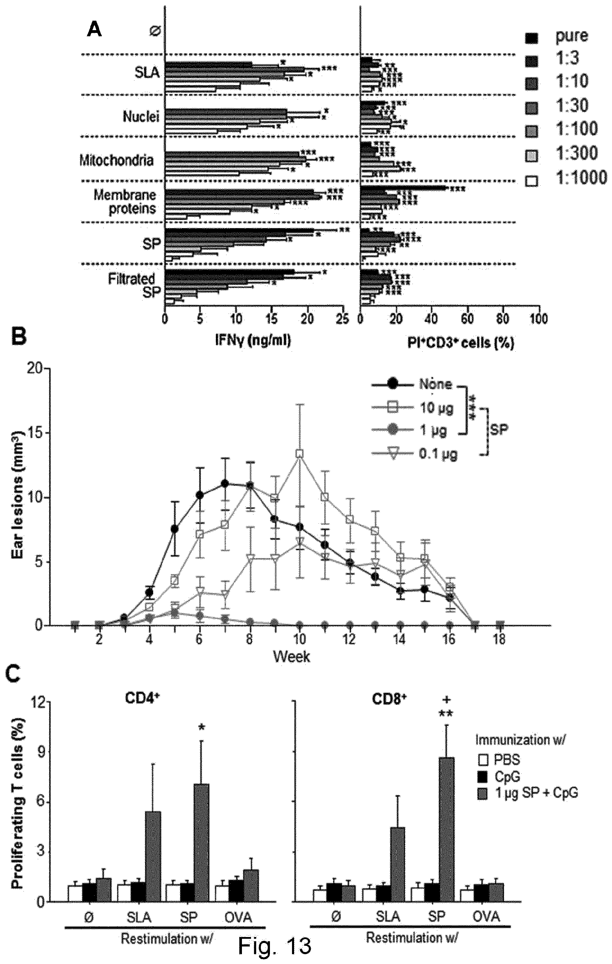

**Fig. 13****: Soluble proteins (SP) from *L. major* lysate are immunogenic and protect against infection. (A)** Soluble *Leihsmania* antigen (SLA) was separated into its components by differential centrifugation. SP were contained in the final supernatant, which was additionally sterile-filtered. Antigen-specific restimulation with primed dLN cells from *L*. *major*-infected C57BL/6 mice was performed with titrated SLA fractions. Induced IFNγ levels and cytotoxicity of CD3⁺ cells *via* PI staining (1 µg/ml; frequency of PI+ CD3⁺ cells minus mean of the unstimulated control) are shown (n=2-3, exemplarily shown for one SLA batch out of two). **(B)** Lesion volume progression of *L. major*-infected C57BL/6 mice immunized i.d. into one ear with different doses of SP combined with CpG as adjuvant (n=3 for 10 µg SP + CpG, n=2 for 1 µg SP + CpG, n=1 for 0.1 µg SP + CpG, ≥4 mice per group per independent experiment). **(C)** C57BL/6 mice were triply injected i.d. with PBS, CpG or 1 µg SP + CpG into one ear. One week later isolated dLN cells were labeled with CFSE (1 µM) and antigen-specific stimulated with SLA, SP (50 µg/ml) or OVA (100 µg/ml) or left unstimulated (Ø). Proliferation rates of CD4⁺ and CD8⁺ T cells were assessed by flow cytometry (n=2 with ≥3 mice per group per experiment). **(A+C)** Unpaired Student's *t* test or **(B)** one-way ANOVA with Bonferroni post-test was used to determine statistical significances. * *p* ≤ 0.05, ** *p* ≤ 0.005, *** *p* ≤ 0.002 (*vs*. unstimulated or CpG control, ⁺P ≤ 0.05 (*vs.* PBS control); mean ± t. dLN, draining lymph nodes, PI, propidium iodide, SEM, standard error of the mean, i.d., intradermally, PBS, phosphate buffered saline, CpG, cytosine phosphodiester guanine oligodeoxynucleotides, *vs*., *versus*, w/, with.

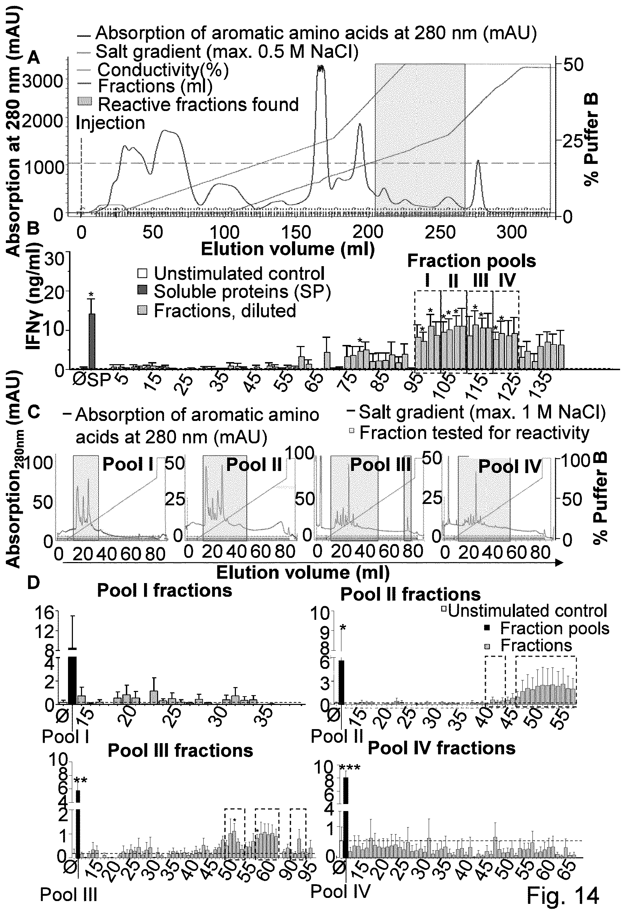

**Fig. 14****: Reactive capacity of SP fractions *in vitro* after two-step anion exchange chromatography. (A)** Anion exchange chromatogram of SP from SLA. 22 ml sterile-filtered SP were applied to the Toyopearls DEAE-605S HR-16/50 column; a linear 1 M NaCl gradient was applied up to 25% followed by a step up to 50% NaCl. The absorption of proteins was monitored at 280 nm and 2 ml fractions were collected. The grey square indicates the fractions chosen for further *in vitro* analyses. **(B)** Induced IFNγ production by 1:3-diluted fractions in an restimulation assay with primed dLN cells from C57BL/6 mice infected with *L*. major parasites (n=3). Pools I-IV were prepared from framed fractions for further chromatographic fractionation. **(C)** Dialysed fraction pools were further separated by a MonoQ 5/50 GL column. A linear 1 M NaCl gradient was applied up to 75% followed by 100% 1 M NaCl, 0.8 ml fractions were eluted. The protein absorption was detected at 280 nm. Marked fractions (grey squares) were analyzed *in vitro.* **(D)** IFNγ levels after antigen-specific restimulation of dLN cells from *L. major-infected* C57BL/6 mice with 'MonoQ'-fractions (n=3-5). Framed fractions (blue) were selected for further analyses. **(B+D)** Statistical significance was determined using unpaired Student's *t* test. * *p* ≤ 0.05, ** *p* ≤ 0.005, *** *p* ≤ 0.002; mean ± SEM. DEAE, diethylaminoethyl cellulose.

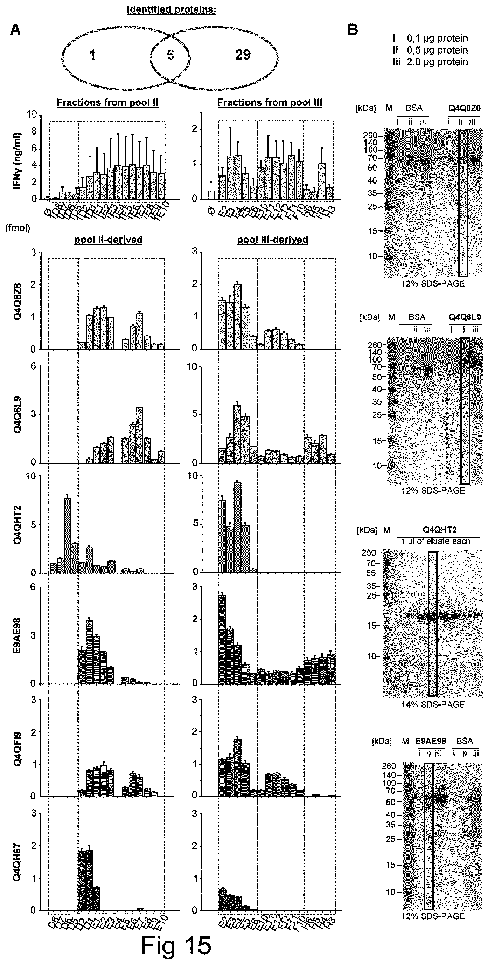

**Fig.** 15: **Identification of** *L*. ***major*-specific proteins by mass spectrometry.** (A) Reactive 'MonoQ'-fractions from pool II and III were analyzed for their protein composition by label-free mass spectrometry (3-6 measurements per fraction; mean ± SEM). The numbers of overall identified *L*. *major*-specific proteins are displayed. The amount of substance (fmol) of 6 single proteins detected in both pool II and III fractions are shown next to the corresponding IFNγ profiles. Yeast enolase-1 served as reference protein for the calculation of the amounts of single proteins. (B) Coomassie stained SDS-PAGEs with 4 purified proteins after recombinant expression in *E. coli* are depicted. Defined amounts of BSA served as control. Gel pictures (Q4Q8Z6, Q4Q6L9 and E9AE98 or Q4QHT2) were kindly provided by Trenzyme GmbH (Constance, Germany) or Danielle Arnold-Schild (University Medical Center, Mainz, Germany, respectively. Recombinant proteins Q4Q8Z6 and Q4Q6L9 were run on the same gel sharing the same marker (dotted line; Figure S3) and for recombinant protein E9AE98 different parts of the gel were assembled (dotted line; Figure S4).

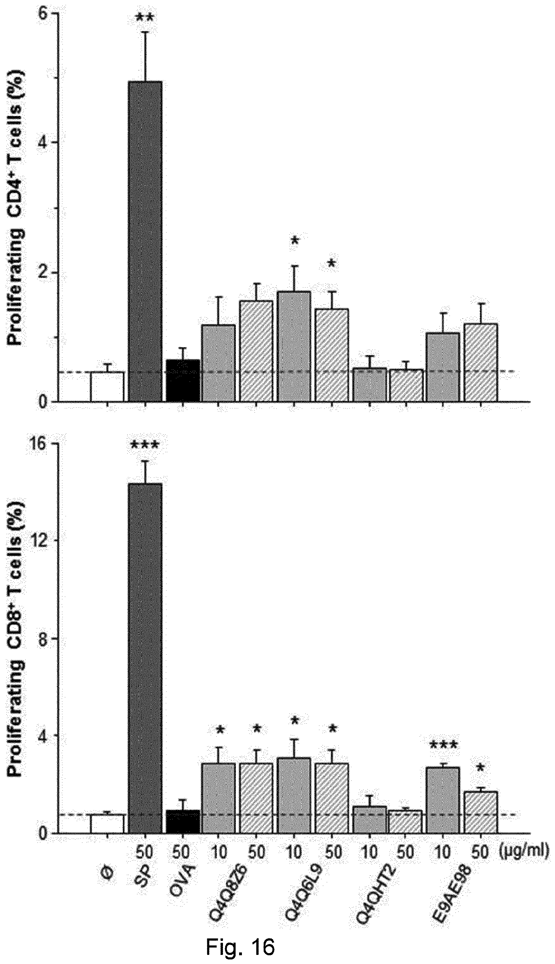

**Fig. 16****: Protein-specific induction of T cell proliferation from primed dLN.** Proliferation frequencies of CD4⁺ and CD8⁺ T cells (pregated for CD3⁺ cells) were displayed after protein-specific restimulation (10 µg/ml or 50 µg/ml) for 5 d of CFSE-labeled (1 µM) dLN cells from C57BL/6 mice with healed *L.major* infection (n=3). The unpaired Student's *t* test was used to determine statistical significance. * *p* ≤ 0.05, *** *p* ≤ 0.002; mean ± SEM.

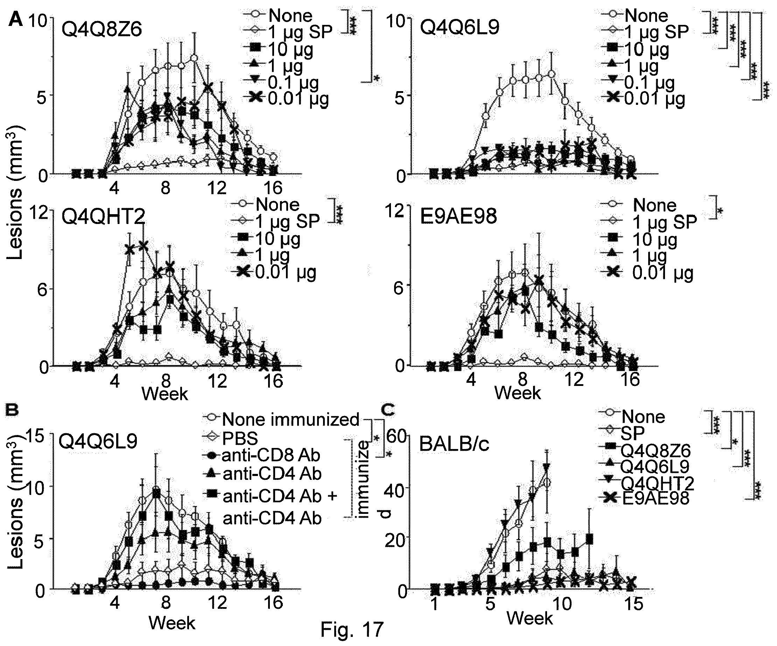

**Fig. 17****: Protein-specific protective capacity against *L. major* infection *in vivo* requires CD4⁺ T cells. (A)** Ear lesion progression after infection with 10³ metacyclic *L. major* promastigotes of C57BL/6 mice triply immunized with different amounts of recombinant proteins with CpG as adjuvant is shown. Treatment before infection was done with Q4Q8Z6 (n=3 independent experiments for 10 µg, n=1 for all other groups), Q4Q6L9 (n=3 for 10 µg, n=2 for 1 µg, n=1 for 0.1 µg and 0.01 µg), Q4QHT2 or E9AE98, respectively (n=2 for 1 µg each, n=1 for 10 µg and 0.01 µg each), plus 10 µg CpG each. **(B)** To determine the dependence of different T cell subsets on the protective effect of Q4Q6L9 (1 µg protein + CpG) *in vivo* in C46BL/6 mice against infection, CD4⁺ or CD8⁺ or both CD4⁺ and CD8⁺ T cells, respectively, were depleted during the immunization period using monoclonal antibodies (anti-CD4, clone GK 1.5, 125 µg/mouse; anti-CD8, clone YTS169, 50 µg/mouse). I.d. infection with 10³ *L. major* parasites was initiated after recurrence of at least 60% of all T cells and developing lesion volumes are shown. (n=2 experiments). **(C)** Developing ear lesions of BALB/c mice triply immunized with 1 µg of recombinant protein combined with CpG as adjuvant (n=2 experiments) after *L. major* infection. Lesion volumes represent mean ± SEM of 3-5 mice per group per independent experiment. Mice treated only with CpG (none) served as negative controls and mice immunized with 1 µg SP + CpG as positive controls. **(A-C)** Statistical analysis was performed using one-way ANOVA with Bonferroni post-test. * *p* ≤ 0.05, *** *p* ≤ 0.002.

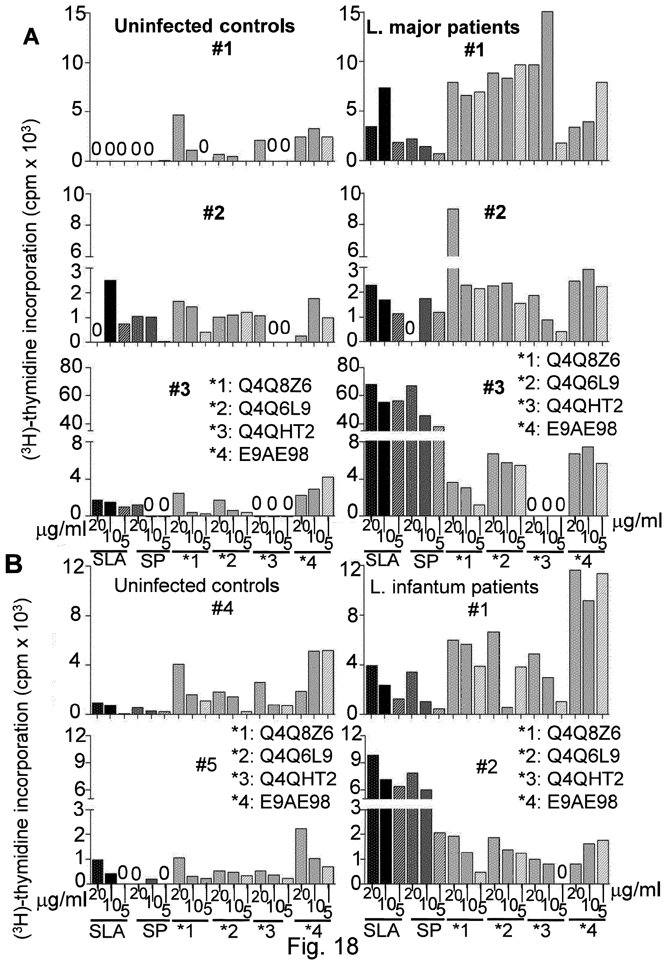

**Fig. 18****: Antigen-specific restimulation of PBMCs from donors with prior *Leishmania* infection.** Isolated PBMCs from patients with prior **(A)** *L.major* (3 donors) or **(B)** *L.infantum* (2 donors) infection, respectively, or unexposed (healthy) controls were stimulated with tetanus toxoid (TT; 10 µg/ml), SLA, SP or recombinant proteins (20, 10, 5 µg/ml) for 5 d and pulsed with (³H)-thymidine (37 kBq/well) for 18 hrs. The uptake of (³H)-thymidine was measured by scintillation counter. Antigen-induced cell proliferation were displayed as bar charts and mean counts per minute (cpm).

## Claims

1. A vaccine comprising a polypeptide or protein selected from the group consisting of *Leishmania major* p54, Q4Q8Z6, Q4Q6L9, Q4QHT2, E9AE98, or an immunologically effective fragment thereof, for use in the prevention or treatment of leishmaniasis.

2. Vaccine according to claim 1, wherein p54 is a MHC class I-restricted polypeptide which comprises an amino acid sequence having at least 90% sequence identity to a polypeptide comprising an amino acid sequence as set forth in SEQ ID NO: 1.

3. Vaccine according to claim 1, wherein Q4Q8Z6 comprises an amino acid sequence having at least 70% sequence identity to a polypeptide or protein comprising an amino acid sequence as set forth in SEQ ID NO: 2.

4. Vaccine according to claim 1, wherein Q4Q6L9 comprises an amino acid sequence having at least 70% sequence identity to a polypeptide or protein comprising an amino acid sequence as set forth in SEQ ID NO: 3.

5. Vaccine according to claim 1, wherein Q4QHT2 comprises an amino acid sequence having at least 70% sequence identity to a polypeptide or protein comprising an amino acid sequence as set forth in SEQ ID NO: 4.

6. Vaccine according to claim 1, wherein E9AE98 comprises an amino acid sequence having at least 70% sequence identity to a polypeptide or protein comprising an amino acid sequence as set forth in SEQ ID NO: 5.

7. Vaccine according to any one of claims 1 to 6, wherein p54, Q4Q8Z6, Q4Q6L9, Q4QHT2, E9AE98, or a combination thereof, is expressed in an gene expression vector.

8. Vaccine according to claim 7, wherein the expression vector contains a polynucleotide sequence encoding a polypeptide having at least 70% sequence identity to a polypeptide comprising the amino acid sequence as set forth in SEQ ID NO: 1, SEQ ID NO: 2, SEQ ID NO: 3, SEQ ID NO: 4, SEQ ID NO: 5.

9. Vaccine according to any one of claims 1 to 8, wherein the p54, Q4Q8Z6, Q4Q6L9, Q4QHT2, E9AE98 is a homologous molecule from another *Leishmania* subspecies.

10. A pharmaceutical composition comprising a vaccine according to any one of claims 1 to 9, and a pharmaceutically acceptable vehicle, diluent, adjuvant or excipient for use as a medicament.

11. A pharmaceutical composition comprising a vaccine according to any one of claims 1 to 9, and a pharmaceutically acceptable vehicle, diluent, adjuvant or excipient for use in the prevention or treatment of leishmaniasis in humans or animals.

12. The pharmaceutical composition according to claim 11, wherein the leishmaniasis is a cutaneous, mucocutaneous or visceral leishmaniasis.

13. The pharmaceutical composition according to claim 11, wherein the cutaneous leishmaniasis is caused by an infection with *L. tropica, L. major, L. aethiopica, L. infantum*, *L. donovani, L. mexicana, L. venezuelensis,* or a subgenus thereof.

14. The pharmaceutical composition according to claim 11, wherein the mucosal leishmaniasis is caused by an infection with *L. viannia* or *L. amazonensis.*

15. The pharmaceutical composition according to claim 11, wherein the visceral leishmaniasis is caused by an infection with *Leishmania donovani* or *Leishmania infantum.*
